# EUROPEAN PATENT APPLICATION

(11) **EP 2 206 776 A1**
(43) Date of publication of application: **14.07.2010**
(21) Application number: 08837299.0
(22) Date of filing: 10.10.2008
(51) Int. Cl.: C12N 15/09, A01H 1/00, A01H 5/00, A23L 1/305, A61K 36/899, A61P 37/08, C07K 14/435, C07K 19/00, C12N 5/10

(54) **MITE ANTIGENIC RICE**

(30) Priority: 12.10.2007 JP 2007266864
(71) Applicant: NATIONAL INSTITUTE OF AGROBIOLOGICAL SCIENCES, Tsukuba-shi Ibaraki 305-8602 (JP)
(72) Inventor: TAKAIWA, Fumio, Tsukuba-shi Ibaraki 305-8602 (JP); SUZUKI, Kazuya, Tsukuba-shi Ibaraki 305-8602 (JP); YANG, Li jun, Tsukuba-shi Ibaraki 305-8602 (JP)
(74) Representative: Plougmann & Vingtoft A/S
(86) International application number: PCT/JP2008/068446
(87) International publication number: WO 2009/048133

(57) **Abstract**

An objective of the present invention is to provide methods for accumulating in rice seeds a partial peptide of a mite antigen protein comprising several T cell epitopes, or a mite antigen peptide that has been modified to not form a conformation to be recognized as an antigen, and plants which have accumulated these peptides.

To achieve the above objective, the present inventors have tried to generate seeds (rice) of rice plants that have accumulated mite antigen peptide variants. As a result, the present inventors developed genetically modified rice plants which express and accumulate these mite antigen peptide variants, and demonstrated their effect on asthma, in particular, by orally feeding mice with these variants to induce immunological tolerance, thereby completing the present invention.

## Description

### Technical Field

The present invention relates to methods for accumulating in rice plants, partial peptides containing major human T cell epitopes in a mite protein, modified peptides with reduced mite antigen-specific IgE binding activity, or peptides that have been modified so that the cysteine protease activity is inactivated, and rice plants that have accumulated such a peptide.

### Background Art

Patients with house dust allergy are present in about 5 to 10% in the world, and in particular, the major cause - allergy caused by feces and dead bodies of mites is a leading cause of bronchial asthma and atopic dermatitis. It not only affects adults but can also be a lethal disease in children. Allergy is a disease caused by Th2 type immune response, in which substances not normally recognized as an antigen are recognized as antigens.

In recent years, a radical treatment for allergic diseases has been hyposensitization therapy in which an allergen *per se* is administered by conventional injection at stepwise increasing doses over a long period of time so as to reduce allergen-specific immunoreactions. However, it has been noted that the allergens used in this therapy retain reactivity with the IgE antibody bound to mast cells which cause allergic symptoms, and therefore may result in problematic side effects such as anaphylactic shock.

Peptide immunotherapy involving the administration of an allergen-derived T-cell epitope peptide has drawn much attention. Its action mechanism is presumed to involve the induction of unresponsiveness or deletion of allergen-specific type 2 helper T cells. Peptide immunotherapy using T-cell epitopes is quite safe because it does not involve B cell epitopes, which cause allergic reactions, or binding to the allergen-specific IgE antibody. As a result, it minimizes the side effects observed in conventional hyposensitization therapy.

Meanwhile, the mechanism known as oral immunological tolerance suppresses the immune system to prevent unnecessary immune responses against orally ingested proteins or such. T cell epitope peptides derived from allergens of cedar pollen allergy also show high reactivity to specific T cells. Thus, the present inventors have previously developed a method for inducing immunological tolerance by accumulating cedar allergen-specific T cell epitopes in practically useful plants and orally administering these plants (Patent Document 1).

A particularly serious allergy in Japan is caused by two types of mites called by *Dermatopagoides farinae* (*D. farina*) and *Dermatopagoides* (*D. pteronyssinus*). There are many reports on the allergenic analysis of these mites, and twenty or more types of allergens have been reported to date (Non-patent Documents 1 to 3). However, of these, about 60 to 90% of the IgE binding activity in mite allergy patients is with groups of proteins belonging to type 1 or type 2 allergens.

The type 1 antigens such as Der p1 and Der f1 are primarily contained in mite feces, and their molecular weights are about 25 kDa. They are known to encode both cysteine protease and serine protease, and the active sites of these two enzymes are the same amino acids (Non-patent Document 4). The cleavage of CD25 (IL-2 receptor α chain), a molecular marker on immunocompetent cell surface, by this cysteine protease activity has been reported to shift the *in vivo* immune response to a Th2-type response (Non-patent Document 5). It is also reported that CD23 (a low-affinity IgE receptor) is similarly cleaved so that the negative feedback signal for IgE production is inhibited (Non-patent Document 6). It is also known to be important for this protein to retain its cysteine protease activity to enter into the body and exhibit its antigenic effect as a mite antigen (Non-patent Document 7).

Meanwhile, type 2 antigens such as Der p2 and Der f2 are proteins with a molecular weight of about 14 kDa, mainly present in mite bodies; however, their functions and roles still remain to be clarified. The conformation is maintained by three sets of intramolecular S-S bonds (Non-patent Document 8), and the conformation has been revealed by NMR analysis (Non-patent Documents 9 and 10). Furthermore, it has been reported that when a mutation is introduced into the S-S bond constituted by cysteines at positions 8 and 119, of the cysteines constituting these S-S bonds, the binding ability of IgE in the serum is markedly reduced whereas the proliferation of antigen-specific T cells is comparable to that of the wild type in human (Non-patent Document 11).

Information of prior art documents related to the present invention is indicated below:
Patent Document 1: WO 2004/094637.
Non-patent Document 1: Thomas, W.R. et al., Int Arch Allergy Immunol 129, 1-18 (2002).
Non-patent Document 2: Kawamoto, S. et al., J Biosci Bioeng 94, 285-298 (2002).
Non-patent Document 3: Weber, E. et al., J Allergy Clin Immunol 112, 79-86 (2003).
Non-patent Document 4: Hewitt C.R. et al., Clin Exp Allergy 27, 201-207 (1997).
Non-patent Document 5: Schulz, O. et al., J Exp Med 187, 271-275 (1998).
Non-patent Document 6: Hewitt, C.R. et al., J Exp Med 182, 1537-1544 (1995).
Non-patent Document 7: Kikuchi Y et al., J Immunol. 177, 1609-1617 (2006).
Non-patent Document 8: Nishiyama C. et al., Int Arch Allergy Immunol 101, 159-166 (1993).
Non-patent Document 9: Ichikawa S. et al., J. Biol Chem 273, 356-360 (1998).
Non-patent Document 10: Mueller G.A. et al., Biochemistry 37, 12707-12714 (1998).
Non-patent Document 11: Takai T. et al., Nature Biotechnol 15, 754-758 (1997).

### Disclosure of the Invention

### [Problems to be Solved by the Invention]

The present invention was achieved in view of the above circumstances. An objective of the present invention is to develop rice plants with accumulated mite antigen peptide variants for application of mite antigen peptide variants as peptide vaccines in the treatment or prevention of mite allergy.

More specifically, an objective of the present invention is to provide methods for accumulating in rice seeds, partial peptides of mite antigen proteins comprising multiple T cell epitopes or mite antigen peptides that have been modified to not form a conformation for antigen recognition; and plants which have accumulated these peptides. Another objective of the present invention is to provide methods for treating or preventing allergic diseases, which comprise the step of orally administering to subjects these rice seeds, or food compositions or pharmaceutical compositions containing these rice seeds.

### [Means for Solving the Problems]

In order to solve the problems described above, the present inventors attempted to accumulate mite antigen peptide variants in rice, which is the seed of rice plants, and induce oral immunological tolerance by ingestion of the rice to treat mite allergy.

Specifically, the present inventors developed rice plants that have accumulated in the endosperm, which is an edible part of rice, a partial peptide containing major human T cell epitopes in a mite protein; a modified peptide that has been modified to have reduced mite antigen-specific IgE-binding activity by keeping T cell epitopes without forming the protein conformation; or a peptide which has been modified such that the cysteine protease activity is inactivated.

In order to highly accumulate these modified mite antigen peptide variants in the rice seed endosperm, the codons of the genes encoding these mite antigen peptide variants were modified to codons that are frequently used in genes of storage proteins in the rice plant. In addition, the glutelin signal peptide was linked to the N terminus, and the endoplasmic reticulum retention signal was attached to the C terminus. The mite antigen peptide variants were confirmed to thereby accumulate in large amounts, at 30 to 80 µg per rice seed (about 20 mg)).

When such epitope peptides of allergens are accumulated at high levels in edible parts, the allergic reaction caused by the allergens can be treated using the immunological tolerance mechanism through oral ingestion. Mice were sensitized by orally administering a mite antigen rice of the present invention for one to four weeks. As a result, it was confirmed that when compared to mice orally administered with non-recombinant rice as a control, mite antigen rice expressing any of the modified peptides was able to reduce mite antigen-specific IgE and IgG levels and induce immunological tolerance. In addition, the numbers of eosinophils and macrophages in respiratory tract tissues were markedly reduced in these mice as compared to those in the control mice. This shows that the respiratory tract resistance to air allergens can also be alleviated by orally administering mite antigen rice of the present invention.

Accordingly, the present inventors have developed genetically modified rice plants that express and accumulate mite antigen peptide variants, and demonstrated their effect against in particular asthma by orally feeding mice with the plants to induce immunological tolerance, thereby completing the present invention.

More specifically, the present invention provides:
[1] a DNA construct having a structure in which the DNA of any one of (a) to (d) below is placed under the control of a seed storage protein promoter of a rice plant:
   (a) a DNA encoding a peptide derived from a mite antigen protein, wherein the DNA comprises a DNA encoding a storage protein signal sequence added at the 5'f end and or a DNA encoding an endoplasmic reticulum retention signal sequence added at the 3' end, and wherein the peptide comprises a plurality of T cell epitopes;
   (b) a DNA encoding a peptide derived from a mite antigen protein, wherein the peptide comprises a storage protein signal sequence added at the N terminus and/or an endoplasmic reticulum retention signal sequence added at the C terminus, and wherein the peptide comprises a plurality of T cell epitopes;
   (c) a DNA encoding a mite antigen protein, wherein the DNA comprises a DNA encoding a storage protein signal sequence added at the 5'f end and or a DNAencod ng an endoplas ṁc reticulum retention signal sequence added at the 3' end, and wherein the mite antigen protein is modified to not form a conformation to be recognized as an antigen; and
   (d) a DNA encoding a mite antigen protein, wherein the protein comprises a storage protein signal sequence added at the N terminus and/or an endoplasmic reticulum retention signal sequence added at the C terminus, and wherein the protein is modified to not form a conformation to be recognized as an antigen;
[2] the DNA construct of [1], wherein the mite antigen protein is a type 1 or type 2 antigen protein;
[3] the DNA construct of [1], wherein the mite antigen protein comprises the amino acid sequence of:
   (i) an amino acid sequence of any one of SEQ ID NO: 2, 4, 6, and 8; or
   (ii) a DNA encoding a protein which comprises an amino acid sequence with a substitution, deletion, insertion, and/or addition of one or more amino acids in the amino acid sequence of any one of SEQ ID NO: 2, 4, 6, and 8;
[4] the DNA construct of [1], wherein the peptide that comprises a plurality of T cell epitopes comprises the region of any one of:
   (i) two or more regions selected from positions 45 to 67, positions 94 to 104, and positions 117 to 143 in the amino acid sequence of SEQ ID NO: 2;
   (ii) two or more regions selected from positions 21 to 49, positions 71 to 100, positions 93 to 108, positions 110 to 131, and positions 197 to 212 in the amino acid sequence of SEQ ID NO: 2;
   (iii) two or more regions selected from positions 11 to 35, positions 87 to 104, and positions 105 to 129 in the amino acid sequence of SEQ ID NO: 4; and
   (iv) two or more regions selected from positions 35 to 50, positions 35 to 60, and positions 87 to 104 in the amino acid sequence of SEQ ID NO: 4;
[5] the DNA construct of [1], wherein the peptide comprising a plurality of T cell epitopes is a partial peptide which has been modified so that the peptide does not form a conformation to be recognized as an antigen, and wherein the peptide is:
   (i) a peptide which comprises an amino acid sequence comprising the region of positions 45 to 144 in the amino acid sequence of SEQ ID NO: 2; or
   (ii) a peptide which comprises an amino acid sequence comprising the region of positions 1 to 144 in the amino acid sequence of SEQ ID NO: 6;
[6] the DNA construct of [1], wherein the peptide comprising a plurality of T cell epitopes is a peptide which has been modified to not have a cysteine protease activity;
[7] the DNA construct of [6], wherein the cysteine residue (Cys) at position 34 in the amino acid sequence of SEQ ID NO: 2 is substituted to an alanine residue (Ala);
[8] the DNA construct of [1], wherein the mite antigen protein modified to not form a conformation to be recognized as an antigen is modified to not have an IgE-inducing active site;
[9] the DNA construct of [8], wherein the mite antigen protein comprises an amino acid sequence in which any one or more of the cysteine residues (Cys) at positions 8, 21, 27, 73, 78, and 119 in the amino acid sequence of SEQ ID NO: 4 or 8 are modified to a serine residue (Ser);
[10] the DNA construct of [9], wherein the mite antigen protein is a partial peptide in which a cysteine residue in a partial peptide comprising the region of positions 11 to 129 in SEQ ID NO: 4 or the region of positions 11 to 129 in SEQ ID NO: 8 has been modified;
[11] the DNA construct of [1], wherein the DNA encoding a mite antigen protein comprises a nucleotide sequence whose codon has been altered to enable its expression in rice endosperm;
[12] the DNA construct of [1] which comprises the nucleotide sequence of any one of:
   (a) a DNA comprising the nucleotide sequence of SEQ ID NO: 9;
   (b) a DNA which hybridizes under stringent conditions to a DNA comprising the nucleotide sequence of SEQ ID NO: 9;
   (c) a DNA encoding a protein comprising the amino acid sequence of SEQ ID NO: 10; and
   (d) a DNA encoding a protein comprising an amino acid sequence with a substitution, deletion, insertion, and/or addition of one or more amino acids in the amino acid sequence of SEQ ID NO: 10;
[13] a polypeptide encoded by the DNA construct of any one of [1] to [12];
[14] the polypeptide of [13], which has been added with a sugar chain and enclosed in protein body I;
[15] a vector carrying the DNA construct of any one of [1] to [12];
[16] a transformed rice plant cell harboring the DNA construct of any one of [1] to [12], or the vector of [15];
[17] a transformed rice plant comprising the transformed rice plant cell of [16], and accumulating a peptide comprising a plurality of T cell epitopes or a peptide which has been modified to not form a conformation to be recognized as an antigen;
[18] the transformed rice plant of [16], wherein the peptide is accumulated in a seed of the rice plant;
[19] a transformed rice plant, which is a progeny or clone of the transformed rice plant of [17];
[20] a breeding material of the transformed rice plant of [17] or [18];
[21] a seed of the transformed rice plant of [15] or [18];
[22] a food composition for treating or preventing an allergic disease wherein the antigen is a mite, which comprises as an active ingredient the transformed rice plant seed of [21];
[23] the food composition of [22], wherein the allergic disease is type I allergy;
[24] a pharmaceutical composition for treating or preventing an allergic disease wherein the antigen is a mite, which comprises as an active ingredient the transformed rice plant seed of [21];
[25] the pharmaceutical composition of [24], wherein the allergic disease is type I allergy;
[26] the pharmaceutical composition of [24] or [25], which is for oral administration;
[27] a method for accumulating in a rice plant a mite antigen protein-derived peptide comprising a plurality of T cell epitopes, wherein the method comprises the steps of:
   (a) obtaining a DNA encoding a peptide comprising a plurality of T cell epitopes;
   (b) adding a DNA encoding a storage protein signal sequence to the 5'f end and or a DNA encoding an endoplasmic reticulum retention signal sequence to the 3'f end of the DNA obtained in (a); and
   (c) expressing the DNA of (b) in a plant under the control of a storage protein promoter;
[28] a method for accumulating in a rice plant a mite antigen protein-derived peptide that has been modified to not form the conformation to be recognized as an antigen, wherein the method comprises the steps of:
   (a) obtaining a DNA encoding a mite antigen protein;
   (b) modifying the DNA obtained in (a) so that the peptide does not form a conformation to be recognized as an antigen;
   (c) adding a DNA encoding a storage protein signal sequence to the 5'f end and or a DNA encoding an endoplasmic reticulum retention signal sequence to the 3'f end or the DNA obtained in (b); and
   (d) expressing the DNA of (c) in a plant under the control of a storage protein promoter;
[29] the method of [27] or [28], wherein the peptide is accumulated in a seed of the rice plant;
[30] a transformed rice plant in which a mite antigen protein-derived peptide comprising a plurality of T cell epitopes and modified to not form a conformation to be recognized as an antigen has been accumulated, wherein the rice plant is produced by the method of any one of [27] to [29];
[31] a method for treating or preventing an allergic disease, wherein the method comprises the step of orally administering the transformed rice plant seed of [21] to a subject; and
[32] a method for inducing immunological tolerance in a patient having an allergic disease, wherein the method comprises the step of orally administering the transformed rice plant seed of [21] to the subject.

### Brief Description of the Drawings

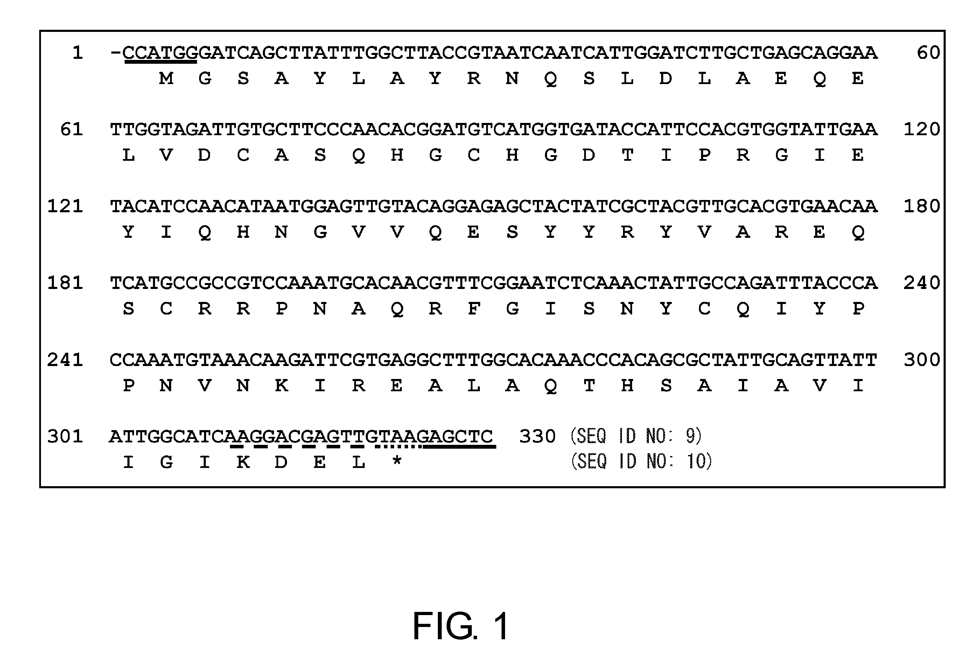

Fig. 1 shows the sequence of a gene synthesized to generate the mite allergen-expressing rice. Upper row: a Der p1-encoding nucleotide sequence (45-145); lower row: the amino acid sequence synthesized as a result of translation of the gene shown in the upper row. An *Nco*I site is attached to the 5'f end (underli nedat the 5'f si de) of the gene sequence, and a sequence encoding KDEL which is a signal for the endoplasmic reticulum (broken line) as well as a stop codon (dotted line) followed by a *Sac*I site (underlined at the 3'f side) are attached to the 3'f end of the gene sequence

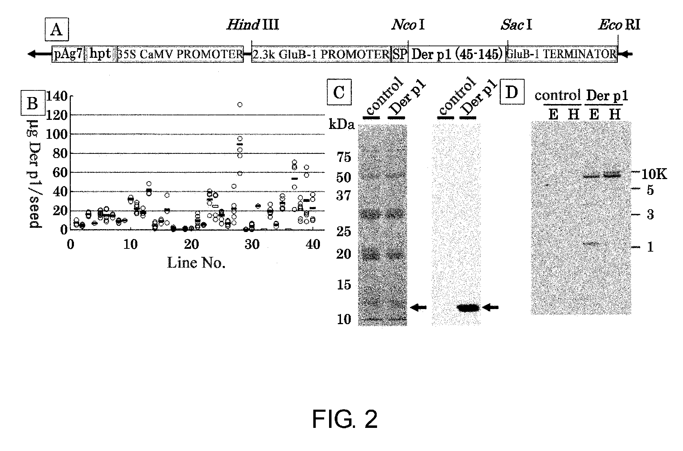

Fig. 2 shows the generation of mite allergen-expressing rice. A shows a construct for mite allergen (Der p1)-expressing rice. B shows the amount of Der p1 accumulated in the seeds of the obtained transformed rice plants. Der p1 in the seeds of each transformant was compared and quantified based on the intensity of color development using an anti-Der p1 antibody, and the No. 28 line was found to have the highest accumulated amount. C shows a result of Der p1 detection by SDS-PAGE and Western blot analysis. A Der p1-specific signal (arrow) was observed by SDS-PAGE (left) and Western blot analysis (right). (Control: non-transformant; Der p1: Der p1-expressing rice transformant) D shows a result of Der p1 gene detection by Southern hybridization. Insertion of the Der p1- encoding gene was only confirmed in the transformant (E: *Eco*RI; H: *Hin*dIII).

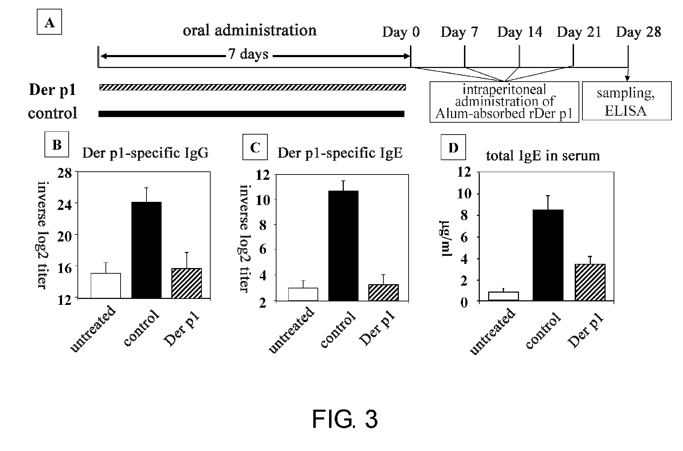

Fig. 3 shows the effect of Der p1-expressing rice in inducing oral immunological tolerance. A shows the schedule for orally administering Der p1 rice to BALB/c mice and immunizing the mice with Der p1 antigen. B shows the amount of Der p1-specific IgG C shows the amount of Der p1-specific IgE; and D shows the total amount of IgE in the serum. It was confirmed from the decreased titer of each antibody that Der p1 rice induced antigen-specific oral immunological tolerance. (Naive (untreated): untreated mice; Control: mice administered with non-transformed rice; Der p1: mice administered with Der p1-expressing rice)

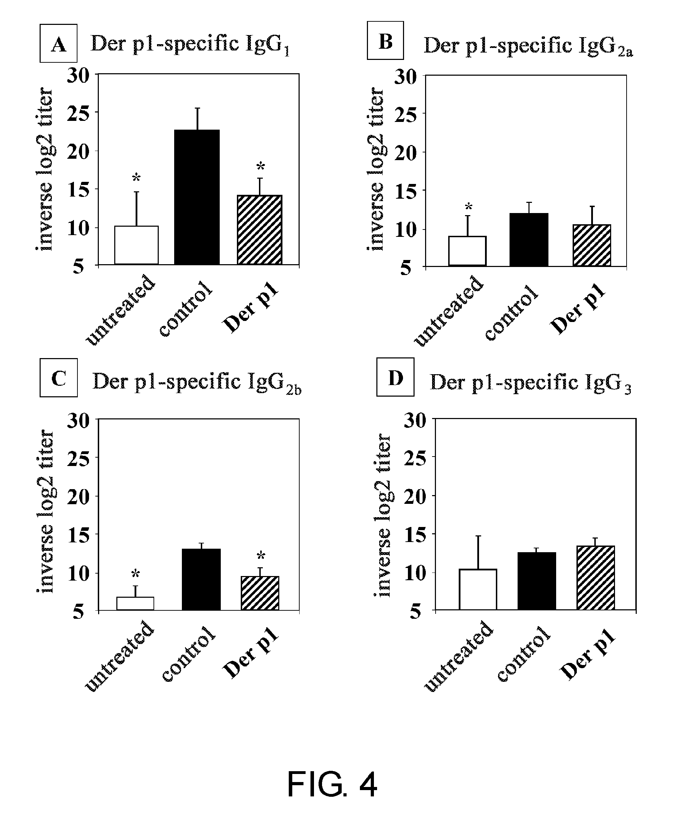

Fig. 4 shows the production of antigen-specific IgG subclasses upon oral administration of Der p1-expressing rice. The results of Der p1-specific IgG₁, Der p1-specific IgG₂ₐ, Der p1-specific IgG_{2b}, and Der p1-specific IgG₃ are shown in A, B, C and D, respectively. The production of antigen-specific IgG₁ and IgG_{2b} was suppressed by oral administration of Der p1 rice.

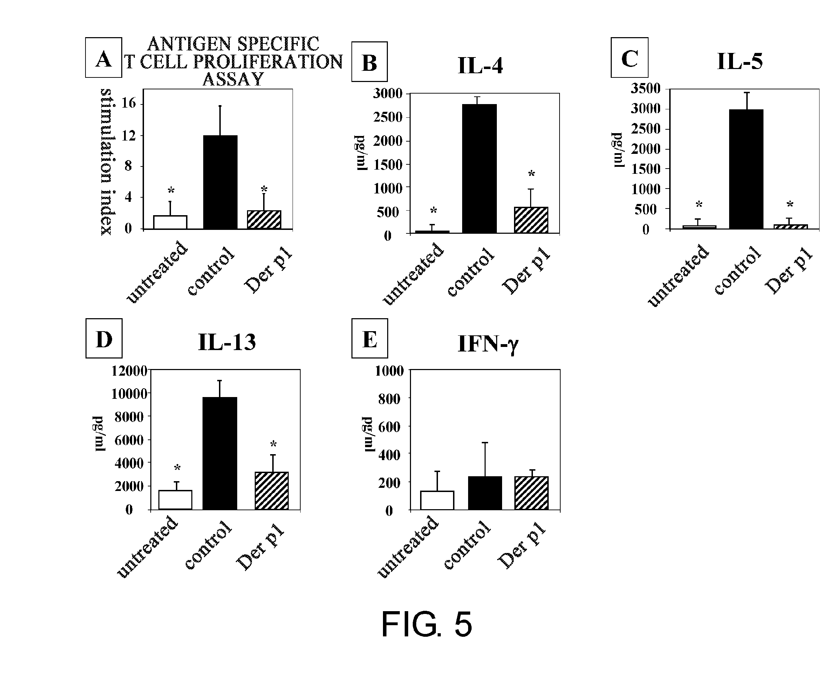

Fig. 5 shows a result of immunological analysis of oral administration of Der p1-expressing rice. A shows Der p1-specific T cell proliferation, and B to E show the secretion of various cytokines. Antigen-specific T cell proliferation was suppressed by oral administration of Der p1 rice. Furthermore, the production of the Th2 cytokines IL-4, -5, and -13 was suppressed, while the production of IFN-γ, a Th1 cytokine, was not altered.

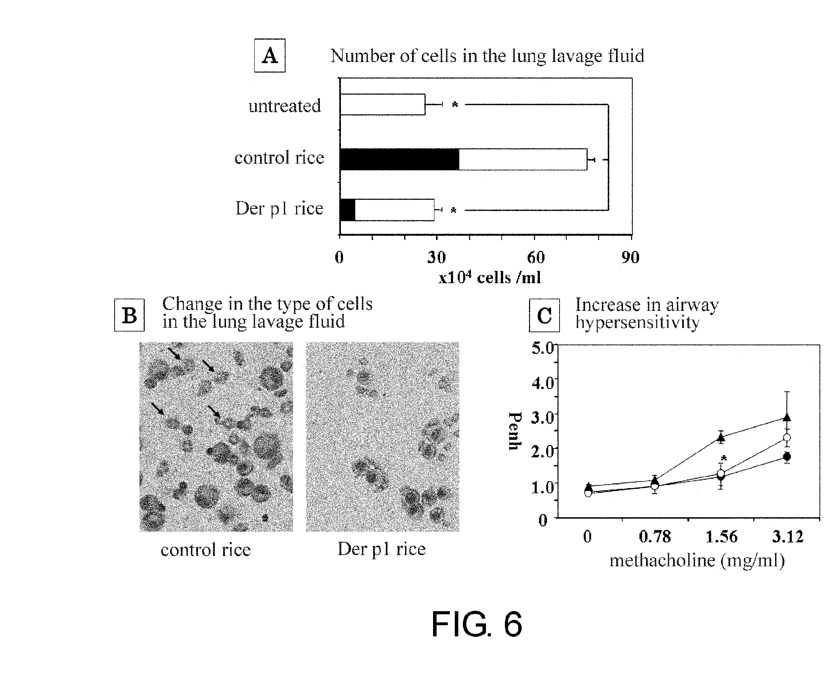

Fig. 6 shows the effect of oral administration of Der p1-expressing rice on airway inflammation. Mice were orally administered with each rice and immunized at the intraperitoneal cavity; and then the antigen was intranasally administered to induce asthma.
   The various symptoms caused by asthma ((A) increase in the number of cells in the bronchoalveolar lavage fluid; (B) infiltration of cells such as macrophages and eosinophils (arrows); (C) airway hypersensitivity) were suppressed by oral administration of Der p1 rice.

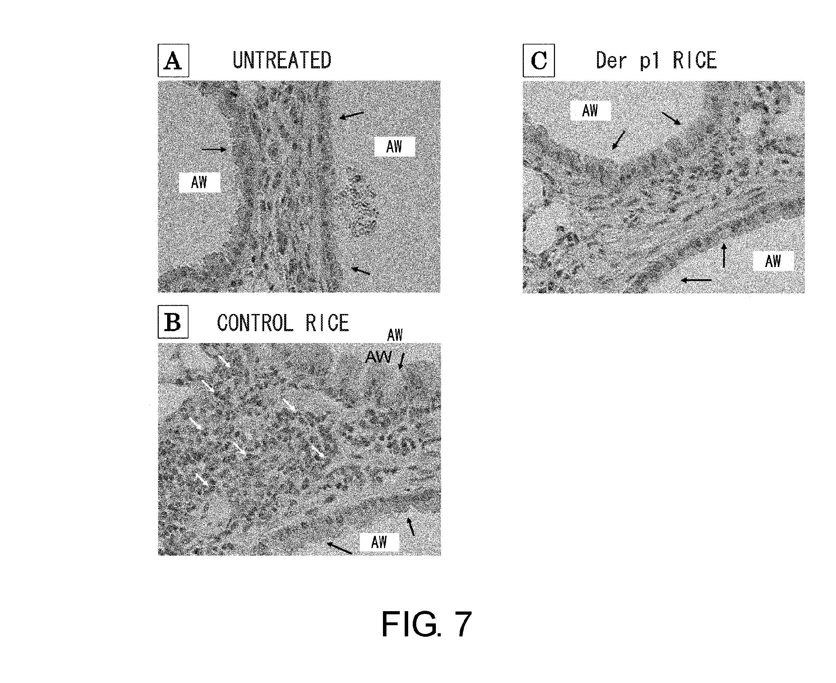

Fig. 7 shows the effect of oral administration of Der p1-expressing rice on lung inflammation. Mice were orally administered with each rice and immunized at the intraperitoneal cavity; and then the antigen was intranasally administered to induce asthma. Lungs were excised from mice of each experimental group and cryosections were prepared. The sections were stained with H and E and the lung conditions of mice in each experimental group were observed. Infiltration of cells such as macrophages and eosinophils (white arrow) and thickening of cells in the airway periphery (black arrow) were observed in the lungs of mice administered with the control rice (B); however, infiltration of these cells and thickening of cells of the epidermis were suppressed in the lungs of mice administered with Der p1 rice (C). (AW: airway)

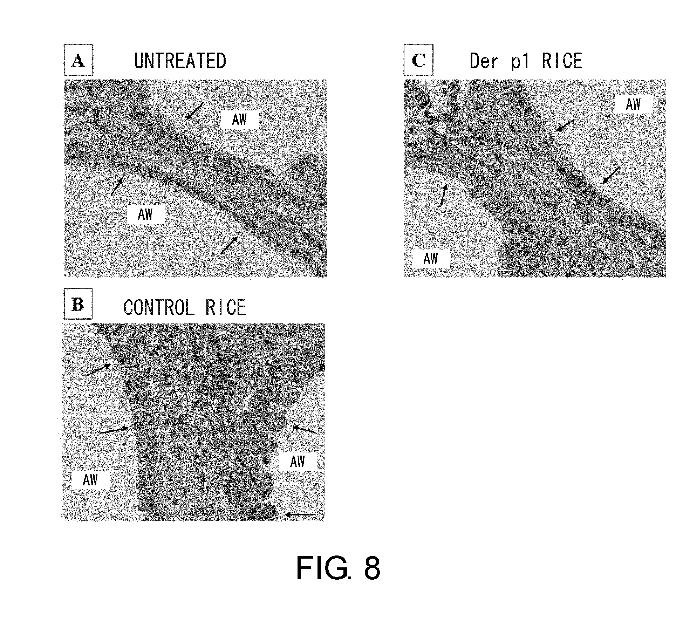

Fig. 8 shows the effect of oral administration of Der p1-expressing rice on lung inflammation. Mice were orally administered with each rice and immunized at the intraperitoneal cavity; and then the antigen was intranasally administered to induce asthma. Lungs were excised from mice of each experimental group and cryosections were prepared. The sections were PAS stained and the lung conditions of mice in each experimental group were observed. Mucus-producing cells in the airway periphery of the lungs of mice administered with the control rice (B) are PAS stained (arrows); however, these cells were not observed in the lungs of mice administered with Der p1 rice (C). (AW: airway)

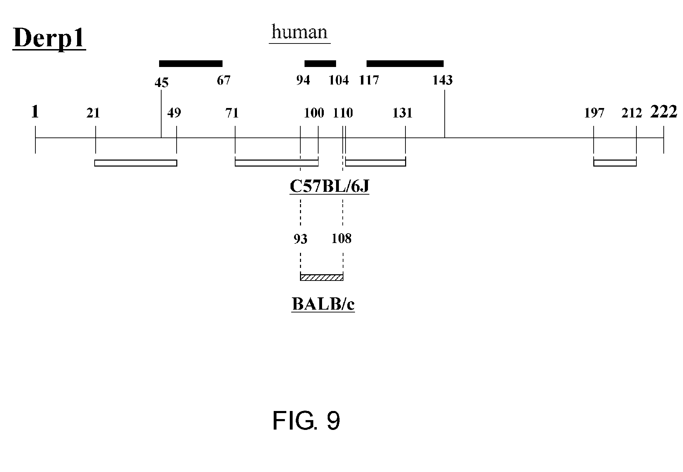

Fig. 9 shows the T cell epitope regions in Der p1. Regions containing the human epitopes and T cell epitopes of the respective mice (C57BL/6J and BALB/c) are shown.

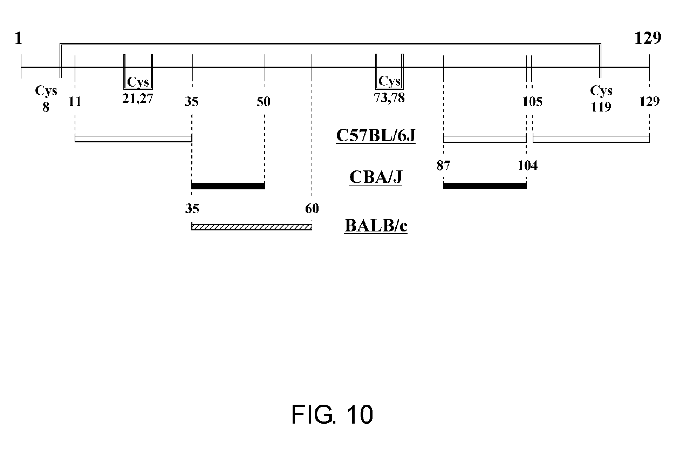

Fig. 10 shows the T cell epitope regions in Der p2. Regions containing the human epitopes and T cell epitopes of the respective mice (C57BL/6J, BALB/c, and CBA/J) are shown.

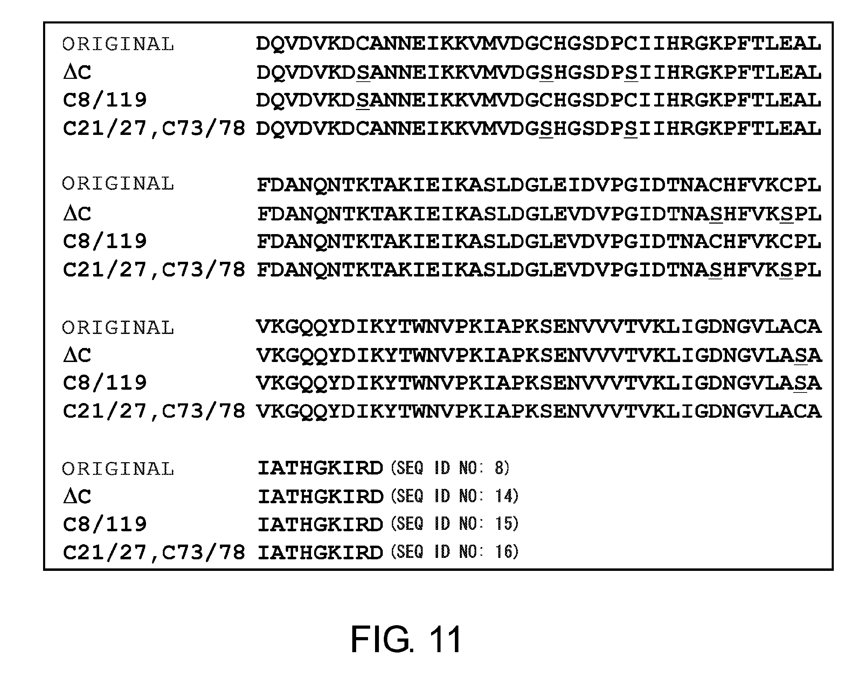

Fig. 11 shows modified Der f2 polypeptides. Underlines indicate mutation sites where a serine residue has been substituted for a cysteine residue.

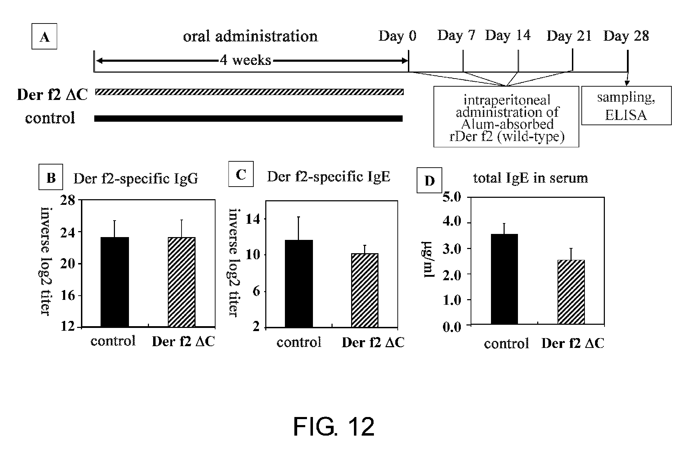

Fig. 12 shows results of induction of oral immunological tolerance by Der f2ΔC-expressing rice. A shows the schedule for orally administering Der f2 ΔC rice to CBA/J mice and immunizing the mice with Der f2 (wild type) antigen. B shows the amount of Der f2-specific IgG; C shows the amount of Der f2-specific IgE; and D shows the total amount of IgE in the serum. There was no significant difference in the amount of antigen-specific IgG and IgE and total IgE in the serum between mice given non-transformed rice and mice given Der f2 ΔC-expressing rice; thus, Der f2 ΔC could not induce immunological tolerance for wild-type Der f2. (Control: mice administered with non-transformed rice plant; Der f2 ΔC: mice administered with Der f2 ΔC-expressing rice).

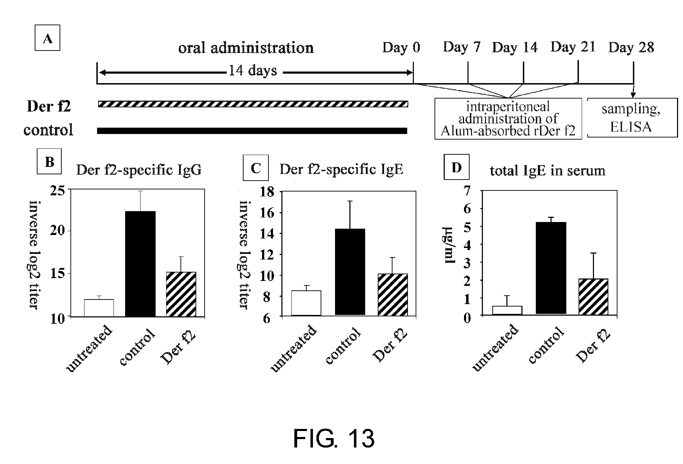

Fig. 13 shows results of induction of oral immunological tolerance by Der f2-expressing rice (a mixture of C8/119 rice and C21/27, C73/78 rice). A shows the schedule for orally administering Der f2 rice to A/J mice and immunizing the mice with Der f2 antigen. B shows the amount of Der f2-specific IgG; C shows the amount of Der f2-specific IgE; and D shows the total amount of IgE in the serum. The titers of the various antibodies were decreased, confirming that Der f2 rice induced antigen-specific oral immunological tolerance. (Naive (untreated): untreated mice; Control: mice administered with non-transformed rice; Der f2: mice administered with Der f2-expressing rice)

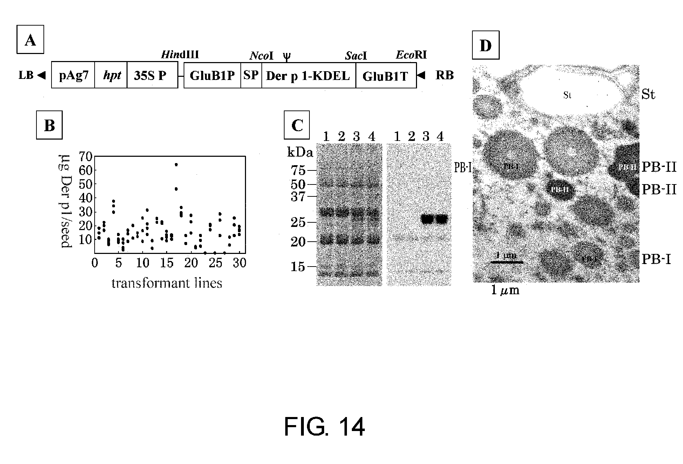

Fig. 14 shows the generation of mite allergen (full-length Der p1)-expressing rice.
   A shows a construct for the mite allergen (full-length Der p1)-expressing rice. B shows the amount of Der p1 accumulated in the seeds of the obtained transformed rice plant. Der p1 in the seeds of each transformant was compared and quantified based on the intensity of color development using an anti-Der p1 antibody, and the No. 17 line was found to have the highest accumulated amount. C shows result of Der p1 detection by SDS-PAGE and Western blot analysis. Der p1-specific signal was observed by SDS-PAGE (left) and Western blot analysis (right). (Lanes 1 and 2: non-transformant; lanes 3 and 4: Der p1-expressing transformant rice). D shows result of Der p1 protein detection in a rice seed by electron microscopy. It was revealed that the Der p1 protein expressed and accumulated in the rice seed was accumulated in protein body I.

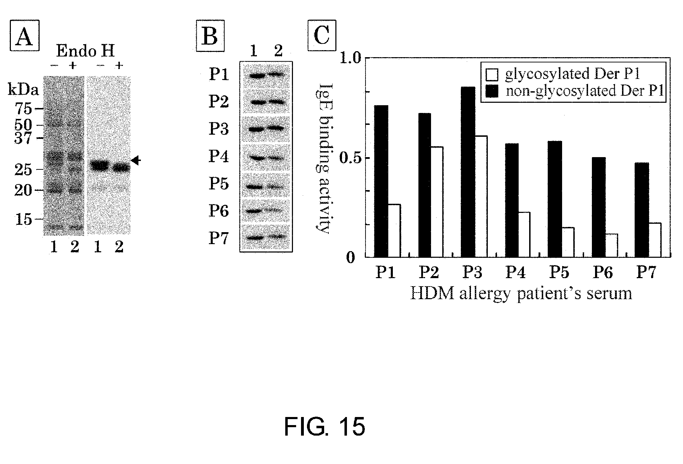

Fig. 15 shows results of analyzing the sugar chains of mite allergen-expressing rice. A shows results of analyzing the sugar chains of mite allergen-expressing rice. Endo H digestion cleaved the sugar chain attached to the Der p1 protein. This demonstrated that the sugar chain attached to Der p1 protein which had accumulated in rice seeds is of the high-mannose type. B and C show results of the reaction between Der p1 protein expressed in rice seeds and serum IgE from mite allergy patients. B: Western blot analysis shows that the reactivity of IgE in the serum of mite allergy patients was reduced by the addition of a sugar chain to the Der p1 protein. (1: Der p1 protein with no sugar chain; 2: Der p1 protein with attached sugar chain) C shows quantitation of the reactivity shown in B. Black bars: Der p1 protein with no sugar chain; white bars: Der p1 protein with attached sugar chain. These findings suggest that Der p1 protein with attached sugar chain causes less of excessive IgE-mediated immune responses.
Fig. 16 shows a modified Der p1 nucleotide sequence. Underlines indicate the sites where the nucleotide sequence encoding the original mite antigen protein was modified to a codon frequently used in the rice plant.
Fig. 17 shows a modified Der f1 nucleotide sequence. Underlines indicate the sites where the nucleotide sequence encoding the original mite antigen protein was modified to a codon frequently used in the rice plant.
Fig. 18 shows a modified Der p2 nucleotide sequence. Underlines indicate the sites where the nucleotide sequence encoding the original mite antigen protein was modified to a codon frequently used in the rice plant.
Fig. 19 shows a Der f2 nucleotide sequence. Underlines indicate the sites where the nucleotide sequence encoding the original mite antigen protein was modified to a codon frequently used in the rice plant.
Fig. 20 shows the result of analyzing the sugar chain of Der p1 protein accumulated in rice seeds.
Fig. 21 shows the result of analyzing the sugar chain of Der p1 protein accumulated in rice seeds.

### Mode for Carrying Out the Invention

The present inventors discovered that by accumulating mite antigen peptide variants in the endosperm, which is an edible part of rice, and orally administering this, the levels of mite antigen-specific IgE and IgG can be decreased and immunological tolerance can be induced. The present invention is based on these findings.

The present invention relates to methods for accumulating mite antigen peptide (allergen) variants in rice seeds.

In general, the term "gllergen" refers to an antigenic substance causing allergic disease (allergic reaction). Allergens suitable for use in the present invention are not particularly limited and include not only naturally-occurring substances, such as proteins and glycoproteins, but also synthetic proteins. Examples of allergens found in nature are pollen (pollens of Japanese cedar, Japanese cypress, alder, ragweed, poaceous cocksfoot, etc.) allergens, animal (dog, cat, mouse, rat, horse, cattle, etc.)-derived allergens, insect allergens, parasite allergens, food allergens, fungal allergens, etc.

Allergens that are suitably used in the present invention are mite antigen proteins. Mite antigen proteins from which the mite antigen peptide variants of the present invention are derived are not particularly limited; however, preferred antigen proteins include type 1 and 2 mite antigen proteins.

Furthermore, the species of mites used in the present invention are not particularly limited, and include, for example, *Dermatophagoides pteronyssinus* and *Dermatophagoides farinae.*

More specifically, mite antigen proteins that are used in the present invention include, for example, Der p1 and Der p2 derived from *Dermatophagoides pteronyssinus,* and Der f1 and Der f2 derived from *Dermatophagoides farinae.* The respective nucleotide sequences and amino acid sequences are as follows:
Der p1 (nucleotide sequence: SEQ ID NO: 1, GenBank accession No. X65197.1; amino acid sequence: SEQ ID NO: 2, type 1 antigen);
Der p2 (nucleotide sequence: SEQ ID NO: 3, GenBank accession No. AM263560.1; amino acid sequence: SEQ ID NO: 4, type 2 antigen);
Der f1 (nucleotide sequence: SEQ ID NO: 5, GenBank accession No. X65196.1; amino acid sequence: SEQ ID NO: 6, type 1 antigen);
Der f2 (nucleotide sequence: SEQ ID NO: 7, GenBank accession No. AY283288; amino acid sequence: SEQ ID NO: 8, type 2 antigen).

Furthermore, the mite antigen proteins used in the present invention include proteins comprising the amino acid sequence with a substitution, deletion, insertion, and/or addition of one or more amino acids in the amino acid sequence of SEQ ID NO: 2, 4, 6, or 8, and which are functionally equivalent to a protein comprising the amino acid sequence of SEQ ID NO: 2, 4, 6, or 8. In addition, a protein encoded by a DNA that hybridizes under stringent conditions to a DNA comprising the nucleotide sequence of SEQ ID NO: 1, 3, 5, or 7, and which is functionally equivalent to a protein comprising the amino acid sequence of SEQ ID NO: 2, 4, 6, or 8 is also included in the mite antigen protein used in the present invention.

In the present invention, the number of amino acids to be mutated is not particularly limited, but is generally 30 amino acids or less, preferably 15 amino acids or less, more preferably five amino acids or less (for example, three amino acids or less). When an amino acid residue is mutated, the amino acid is preferably mutated to a different amino acid which retains the properties of the amino acid side chain (this is known as conservative amino acid substitution). Amino acid side chain properties are roughly divided, for example, into two groups: hydrophobic amino acids (A, I, L, M, F, P, W, Y, and V) and hydrophilic amino acids (R, D, N, C, E, Q, G, H, K, S, and T). Alternatively, amino acids can be categorized according to their side chain structures, for example, into amino acids having aliphatic side chains (G, A, V, L, I, and P), amino acids having hydroxyl group-containing side chains (S, T, and Y), amino acids having sulfur-containing side chains (C and M), amino acids having carboxylic acid- and amide-containing side chains (D, N, E, and Q), amino acids having basic side chains (R, K, and H), and amino acids having aromatic side chains (H, F, Y, and W). Furthermore, classification of amino acids using, for example, a mutational matrix is also known (Taylor 1986, J. Theor. Biol. 119, 205-218; Sambrook, J. et al., Molecular Cloning 3rd ed. A7.6-A7.9, Cold Spring Harbor Lab. Press, 2001). This classification is summarized as follows: aliphatic amino acids (L, I, and V), aromatic amino acids (H, W, Y, and F), charged amino acids (D, E, R, K, and H), positively charged amino acids (R, K, and H), negatively charged amino acids (D and E), hydrophobic amino acids (H, W, Y, F, M, L, I, V, C, A, G, T, and K), polar amino acids (T, S, N, D, E, Q, R, K, H, W, and Y), small amino acids (P, V, C, A, G, T, S, N, and D), very small amino acids (A, G, and S), and large (non-small) amino acids (Q, E, R, K, H, W, Y, F, M, L, and I) (amino acids are represented by one-letter codes in parentheses).

It is already known that a polypeptide having an amino acid sequence modified by a deletion or addition of one or more amino acids and/or substitution with other amino acid(s) retains its biological activity. Furthermore, target amino acid residues are preferably substituted with amino acid residues having as many common properties as possible.

Herein, "g functionally equivalen" h means that subject protein has biological or biochemical functions that are equivalent to those of a mite antigen protein. Biological properties also include specificity for the expression site and expression level.

Methods well known to those skilled in the art for isolating homologous genes include hybridization techniques (Southern, E. M., Journal of Molecular Biology, Vol. 98, 503, 1975) and polymerase chain reaction (PCR) techniques (Saiki, R. K., et al. Science, vol. 230, 1350-1354, 1985, Saiki, R. K. et al. Science, vol.239, 487-491, 1988). Specifically, those skilled in the art can commonly isolate genes homologous to a mite antigen gene from various insects (preferably mites) using as a probe a nucleotide sequence encoding a mite antigen protein (for example, DNA of SEQ ID NO: 1, 3, 5, or 7) or a portion thereof, or using as primers oligonucleotides that specifically hybridize to a mite antigen gene.

In order to isolate DNAs encoding such homologous genes, in general, a hybridization reaction is carried out under stringent conditions. Those skilled in the art can appropriately select stringent hybridization conditions. For example, pre-hybridization is carried out in a hybridization solution containing 25% formamide or 50% formamide under more stringent conditions, and 4x SSC, 50 mM Hepes (pH7.0), 10x Denhardt'fs s d uti on, and 20µg/ml denatured salmon sperm DNA at 42 CC over righ; then labeled probes are added, and hybridization is carried out by incubation at 42 CC over right. Pos-hybridization washing can be carried out with the following conditions for the wash solution and temperature: "glx SSC, 0 1 % SDS, 37 C" or so; "g0 5x SSC, 0 1 % SDS, 42 " or so for a more stringent condition; and "g0 2x SSC, 0.1% SDS, 65 C" or so for an even more stringent condition. As the stringency of the hybridization washes increases, isolation of DNAs with high homology to the probe sequence is expected. However, the above-described combinations of SSC, SDS, and temperature conditions are mere examples, and those skilled in the art can achieve similar stringencies as those described above by appropriately combining the above or other elements (such as probe concentration, probe length, or hybridization reaction time) which determine the stringency of hybridization.

Homology of isolated DNAs indicates a sequence identity of at least 50% or more, more preferably 70% or more, still more preferably 90% or more (for example, 95%, 96%, 97%, 98%, or 99% or more) over the entire amino acid sequence. Sequence homology can be determined using the program of BLASTN (nucleic acid level) or BLASTX (amino acid level) (Altschul et al. J. Mol. Biol., 215: 403-410, 1990). The programs are based on the algorithm BLAST by Karlin and Altschul (Proc. Natl. Acad. Sci. USA, 87:2264-2268, 1990; Proc. Natl. Acad. Sci. USA, 90: 5873-5877, 1993). When analyzing a nucleotide sequence by BLASTN, parameters are set to, for example, score = 100 and wordlength = 12. When analyzing an amino acid sequence by BLASTX, parameters are set to, for example, score = 50 and wordlength = 3. Alternatively, an amino acid sequence can be analyzed using the Gapped BLAST program as indicated by Altschul et al. (Nucleic Acids Res. 25: 3389-3402, 1997). When the BLAST and Gapped BLAST programs are used, the default parameters of each program are used. The specific procedures of these analysis methods are known.

A first embodiment of the mite antigen peptide variants of the present invention includes T cell epitope-containing peptides derived from a mite antigen protein.

Typically, a T cell antigenic determinant (herein, sometimes referred to as "epitope"h or "gepitope peptid" his an antigen peptide displayed on the cell surface following degradation (antigen processing) of an above allergen by antigen-presenting cells, or a partial region of this peptide. Therefore, the amino acid sequences of the epitopes of the present invention are not particularly limited, as long as they are peptides that T cell receptors can recognize as an allergen-derived antigen peptide.

The T cell epitopes contained in a mite antigen peptide variant of the present invention are not particularly limited; however, they are preferably human T cell epitopes.

It is difficult to specify the epitopes (epitope peptides) of the present invention because their peptide length varies depending on the type of allergen or the like. However, typically, they comprise about 10 to 25 amino acid residues, and more preferably 12 to 19 amino acid residues. Epitopes that are used in the present invention may comprise known epitopes (Hoyne et al., (1996) Clin. Immunol. Immunopathol. 80, S23-S30).

Because recognized epitopes differ according to the individual's genotype, when using epitopes in peptide immunization, several epitopes are preferably used in the present invention to obtain an effect in many individuals.

Specifically, the peptides of the present invention which comprise T cell epitopes include, for example, the partial peptides described in (i) to (iv) below, but are not limited thereto:
(i) a region selected from positions 45 to 67, positions 94 to 104, and positions 117 to 143 in the amino acid sequence of SEQ ID NO: 2 (Fig. 9);
(ii) a region selected from positions 21 to 49, positions 71 to 100, positions 93 to 108, positions 110 to 131, and positions 197 to 212 in the amino acid sequence of SEQ ID NO: 2 (Fig. 9);
(iii) a region selected from positions 11 to 35, positions 87 to 104, and positions 105 to 129 in the amino acid sequence of SEQ ID NO: 4 (Fig. 10); and
(iv) a region selected from positions 35 to 50, positions 35 to 60, and positions 87 to 104 in the amino acid sequence of SEQ ID NO: 4 (Fig. 10).

Furthermore, the mite antigen protein-derived peptides of the present invention comprising T cell epitopes are preferably partial peptides which are portions of the full-length mite antigen protein, and more preferably are partial peptides comprising a portion selected to not retain the reactivity to IgE antibodies which bind to allergic symptom-causing mast cells.

Specifically, such partial peptides include, for example, those listed in (i) to (ii) below, but are not limited thereto:
(i) a peptide which comprises an amino acid sequence comprising the region from positions 45 to 144 in the amino acid sequence of SEQ ID NO: 2; and
(ii) a peptide which comprises an amino acid sequence comprising the region from positions 1 to 144 in the amino acid sequence of SEQ ID NO: 6.

Another embodiment of the T cell epitope-comprising mite antigen protein-derived peptides of the present invention includes peptide variants prepared by further modifying the peptides so that they do not have any cysteine protease activity. Der p1 and Der f1 have the cysteine protease activity (Der p1: Stewart, G.A. et al. Int. Arch. Allergy Appl. Immunol. 95: 248-256 (1991); Der f1: Ando, T. et al. Int. Arch. Allergy Appl. Immunol. 96: 199-205 (1991)). When an antigen protein is expressed and accumulated in rice seeds while retaining its enzymatic activity, the enzymatic activity may have adverse effects in the human body after oral ingestion. The following effects are known. For example, the cysteine protease activity of Der p1 increases antigen permeability in the respiratory tract (Kalsheker, N. A. et al. Biochem Biophys Res Commun 221, 59-61 (1996)); Der p1 inactivates α1-antitrypsin (Hewitt, C.R. et al. J Exp Med 182, 1537-1544 (1995)), thereby facilitating the passage of antigens and causing asthma; Der p1 shifts the *in vivo* immune response to a Th2 type response by cleaving CD25 which is present on the surface of immunocompetent cells (Schulz, O. et al. J Exp Med 187, 271-275 (1998)); Der p1 inhibits the negative feedback signal of IgE synthesis by cleaving CD23 (a low-affinity IgE receptor) which is present on cell surfaces (Hewitt, C. R. et al. J Exp Med 182, 1537-1544 (1995)), etc.

The present invention provides peptide variants of mite antigen protein comprising T cell epitopes, which are prepared by further modifying the peptides to not have any of these cysteine protease activities. This enables safe administration of the peptide variants to subjects.

Those skilled in the art can generate such peptide variants by introducing mutations and such into amino acids that are essential for retaining the cysteine protease activity based on information described in references (for example, K.Y Chua, et al. J. Exp. Med. 167 (1988) 175-182) or the like. Specifically, such peptides include, for example, peptide variants in which the cysteine residue (Cys) at position 34 of the amino acid sequence of SEQ ID NO: 2 has been modified to an alanine residue (Ala), but are not limited thereto.

A second embodiment of the mite antigen peptide variants of the present invention includes peptide variants that have been modified to not form a conformation required for recognition as a mite antigen protein.

The conformation of a protein is maintained by various forms of binding such as hydrophobic interaction, hydrogen bond, ionic bond, and disulfide bond. On the other hand, when a mite antigen peptide retains the conformation as an allergen, it retains the reactivity to IgE antibodies which bind to allergic symptom-causing mast cells, and therefore may cause symptoms such as anaphylactic shock. Accordingly, the mite antigen peptide variants of the present invention are preferably modified to not retain the conformation as an allergen. In other words, they are preferably modified to not have any IgE-inducing active center.

Those skilled in the art can routinely use structural information predicted from the amino acid sequence to generate mite antigen proteins that do not form the conformation by modifying mite antigen proteins so that the bonding required for formation of the conformation is inhibited. For example, cysteine residues may be modified to serine residues to inhibit sulfide bond formation (Takai T et al., (1997) Nat Biotechnol vol.15, 754-758).

Specifically, such peptide variants of the present invention which have been modified to not form the conformation include, for example, peptide variants in which one or more of the cysteine residues (Cys) at positions 8, 21, 27, 73, 78, and 119 in the amino acid sequence of SEQ ID NO: 4 or 8 have been modified to a serine residue (Ser). The cysteine residues to be mutated and the combination thereof are not particularly limited; however, the particularly preferred examples in the present invention include those in which the cysteine residues at positions 8 and 119 have been modified, and peptides in which the cysteine residues at positions 21, 27, 73, and 78 have been modified (SEQ ID NOs: 15 and 16, Fig. 11).

In the present invention, mite antigen rice expressing the peptide variants described above can be used by appropriately combining with rice that have been transformed with peptide variants modified at different cysteine residues.

Alternatively, the above variants may be those in which amino acid mutations have been introduced into a partial peptide of the mite antigen protein. Examples include partial peptides in which the cysteine residues in a partial peptide comprising the region of positions 11 to 129 in SEQ ID NO: 4 or a partial peptide comprising the region of positions 11 to 129 in SEQ ID NO: 8 have been modified.

A characteristic of the mite antigen peptide variants of the present invention is that when they are expressed in rice seeds, sugar chains are attached and they are enclosed in protein body I. The attached sugar chains are those with a structure and composition similar to sugar chains attached *in vivo* to proteins in mammals, particularly in humans. Such proteins with added sugar chains are expected to be highly safe when administered orally, because antibodies against the sugar chains are less likely to be produced as compared to proteins with no added sugar chains or with sugar chains attached in other species. Such sugar chains include, for example, those described in the Examples. Information in the references, for example, K. Y. Chua, et al. J. Exp. Med. 167 (1988) 175-182, can be referred to for amino acids that such sugar chains attach.

In a preferred embodiment of the method of the present invention, the mite antigen peptide variants of the present invention are expressed in the rice plant under the control (regulation) of a storage protein promoter. More specifically, first, DNA encoding a mite antigen peptide variant is obtained (process (a)), and then, DNA encoding a storage protein signal sequence is added to the 5'-end and/or DNA encoding ER-retention signal sequence is added to the 3'-end of the DNA obtained in the process (a) (process (b)). Subsequently, DNA obtained in the above process (b) is expressed under the control of the storage protein promoter in a rice plant (process (c)).

Herein, the term "storage protein" generally refers to a protein stored mainly as an energy source in the seeds of a plant. Examples of storage proteins are simple proteins, such as glutelin and prolamin. In the context of the present invention, a preferred storage protein is glutelin. The GenBank accession number for the glutelin-encoding gene is X54314 (O. sativa GluB-1 gene for glutelin) and that the accession number for the cDNA of the gene is X05664.

In the present invention, promoters known in the art can be appropriately selected or modified to be used by one skilled in the art according to the type of genes to be expressed and the types of cells to be transduced. In a preferred embodiment of the present invention, a storage protein promoter can be used. One example of a storage protein promoter suitable for use in the context of the present invention is the glutelin GluB-1 promoter. This promoter is usually not less than 1.3 kb in length, preferably not less than 2.3 kb, but is not particularly limited to this length as long as it is functionally equivalent to the promoter of the present invention. Preferably, the promoter is a 2.3 k GluB-1 promoter. Usually, the longer the promoter is, the higher the expression/accumulation efficiency of a protein encoded by the gene downstream of the promoter becomes. Because of its powerful promoter activity, the glutelin GluB-1 promoter can be suitably used for rice and other grains.

Suitable promoters useful in the methods of the present invention include, besides the above-described glutelin GluB-1, for example, the glutelin GluB-4 promoter, the 10 kD prolamin promoter, and the 16 kD prolamin promoter. Information on nucleotide sequences for the promoters can be acquired as needed from patent or scientific references, or from public databases, such as GenBank (Japanese Patent Application *Kokai* Publication No. 2005-130833; Plant Biotechnology Journal 2, 113-125 (2004) L.Q. Qu and F. Takaiwa, Evaluation of tissue specificity and expression strength of rice seed component gene promoters in transgenic rice; Glub-4 (Accession No.: AY427571); 10 k prolamin (Accession No.: AY427572); 16 kD prolamin (Accession No.: AY427574)).

Examples of DNA encoding a mite antigen peptide variant in the above-described process (a) include genomic DNA, cDNA, and chemically synthesized DNA. Genomic DNA and cDNA from mites, which are the source of allergens, can be prepared by one skilled in the art using conventional methods. Genomic DNA can be prepared, for example, by extracting genomic DNA from mites as a source of allergen, preparing a genomic library (*e.g.*, plasmid, phage, cosmid, BAC, PAC, or the like can be used as the vector), and developing it to perform colony hybridization or plaque hybridization using a probe prepared based on information of the nucleotide sequence of DNA encoding the mite antigen peptide variant. Genomic DNA can also be prepared by performing PCR using specific primers for DNA encoding the mite antigen peptide variant of the present invention. Further, cDNA can be prepared, for example, by synthesizing cDNA based on mRNA extracted from mites as a source of allergen, inserting this cDNA into a vector, such as λZAAP to make cDNA library, and developing it to perform colony hybridization or plaque hybridization, or by PCR, as described above.

It is also possible to appropriately synthesize DNA encoding the mite antigen peptide variant of the present invention artificially, preferably based on information of the amino acid sequence of the peptide. In this case, the objective DNA can be synthesized with reference to codons frequently used in storage protein genes, taking the degeneracy of amino acids and such into consideration. Further, when a mite antigen peptide variant of the present invention is used, a DNA sequence encoding the peptide can be prepared using codons used in the rice seed storage protein gene in high frequency so as to be efficiently translated in rice seeds.

Codons preferably used in the present invention include, but are not particularly limited to, for example, the codons in Table 1.

**Table 1**

| Ala(A) | Asp(D) | Arg(R) | Asn(N) | Cys(C) |
|---|---|---|---|---|
| GCA | GAT | CGT | AAT | TGC |
| GCT | | AGG | AAC | TGT |
| | | AGA | | |
| | | | | |
| Gly(G) | Glu(E) | Gln(Q) | Leu(L) | STOP CODON |
| GGC | GAA | CAA | CTC | TAA |
| GGA | GAG | CAG | CTT | TGA |
| | | | CTA | TAG |
| | | | TTG | |
| | | | | |
| His(H) | Ile(I) | Phe(F) | Pro(P) | Thr(T) |
| CAT | ATC | TTC | CCA | ACT |
| CAC | ATT | | CCC | ACA |
| | | | CCT | ACC |
| | | | | |
| Lys(K) | Ser(S) | Trp(W) | Tyr(Y) | Val(V) |
| AAG | AGT | TGG | TAC | GTT |
| | TCT | | TAT | GTA |
| | AGC | | | |
| | | | | |
| Met(M) | | | | |
| ATG | | | | |

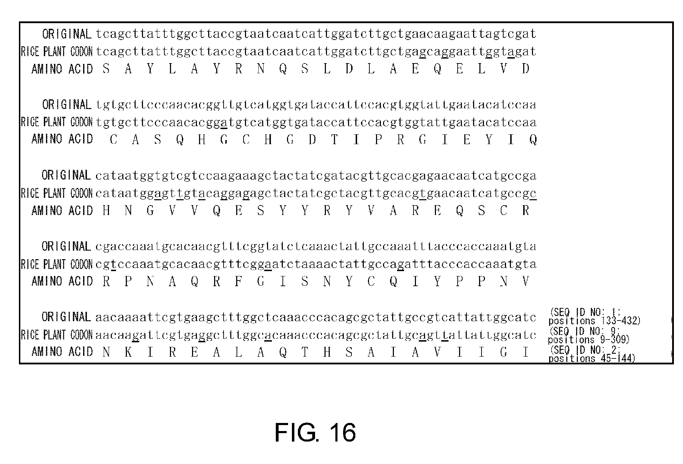

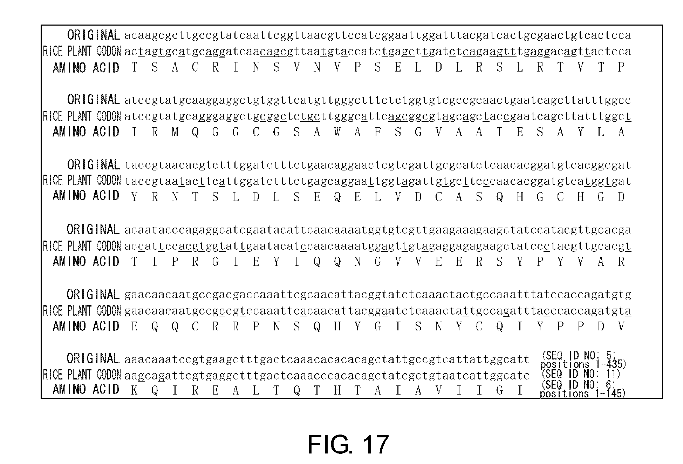

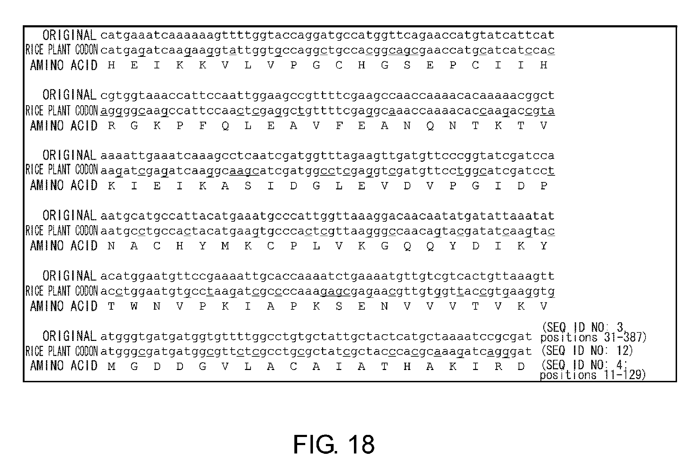

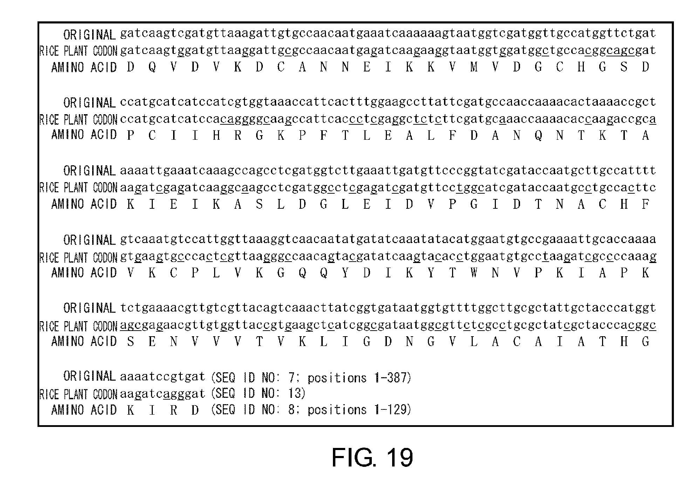

Specifically, such DNA encoding a mite antigen peptide variant with codons modified to those used in high frequency in rice seed storage protein genes include, for example, Der p1 (Fig. 16, SEQ ID NO: 9), Der f1 (Fig. 17, SEQ ID NO: 10), Der p2 (Fig. 18, SEQ ID NO: 12), and Der f2 (Fig. 19, SEQ ID NO: 13).

One skilled in the art can appropriately perform DNA synthesis using a commercial DNA synthesizer or the like.

Regarding the storage protein signal (peptide) sequence in the above-described process (b), various known storage protein signal sequences can be appropriately used. Information on amino acid sequences of storage protein signal sequences of the present invention can be readily obtained by one skilled in the art from known references and the like. Storage protein signals function to locate the mite antigen peptide variant of the present invention to endoplasmic reticulum, thereby preventing the peptide from being located to cytoplasm and degraded or secreted to the outside of the cell.

Regarding the storage protein signal sequence of the present invention, the signal sequence of glutelin (GluB-1) protein can be preferably used. Specifically, storage protein signal sequences usable in the present invention include the following sequence:
MASSVFSRFSIYFCVLLLCHGSMA (SEQ ID NO: 17).

It is also possible to use the signal sequence of another glutelin (GluA-2): MASINRPIVFFTVCLFLLCDGSLA (SEQ ID NO: 18) or that of the 26 kD globulin:
MASKVVFFAAALMAAMVAISGAQ (SEQ ID NO: 19).

Further, regarding the ER-retention signal sequence of the present invention, for example, the KDEL sequence (SEQ ID NO: 20), the SEKDEL sequence (SEQ ID NO: 21), or the HDEL sequence (SEQ ID NO: 22) can be used; however, the present invention is not particularly limited to these examples. DNA encoding the ER-retention signal sequence of the present invention may contain, for example, a 3'-untranslated region downstream of a DNA encoding the KDEL sequence. Although this 3'-untranslated region is not particularly limited, it is usually about 100 to 1000 bp long. As an example, a DNA encoding an ER-retention signal sequence of the present invention is a DNA comprising a DNA encoding the KDEL sequence and a region including the glutelin 3'-untranslated region of about 650 bp in length downstream of that DNA. In general, as the above-described 3'-untranslated region, the 3'-untranslated regions of genes of storage proteins, such as glutelin, can be suitably used. The NOS terminator or the 35S CaMV terminator can also be used. The aforementioned sequences function to improve the amount of foreign proteins accumulated in storage parts of seeds and such.

DNA encoding the above-described storage protein signal sequence or ER-retention signal sequence can be obtained (synthesized) by one skilled in the art by appropriately using a commercial DNA synthesizer or the like, taking the degeneracy of amino acid sequences and such into consideration.

Further, the addition of DNA encoding a storage protein signal sequence to the 5'-end, and DNA encoding an ER-retention signal sequence to the 3'-end of DNA obtained in process (a) can be performed by one skilled in the art using known genetic engineering techniques. The aforementioned "5'-end" or "3'-end" is usually defined as the end to indicate that the direction receiving the transcription control from the promoter is 5'-end -> 3'-end. Accordingly, the DNA end on the promoter side (direction) is defined as the "5'-end."

In the above process (c), generally DNA can be expressed under the control of a storage protein promoter in a plant body by introducing DNA to which the storage protein promoter is linked to express the DNA in the plant body. One skilled in the art can readily locate the DNA to be expressed downstream of the promoter to receive the promoter control, using general genetic engineering techniques.

In the methods of the present invention, the site in the rice plant where the mite antigen peptide variants are accumulated is the seed (rice) which is the edible part, and more specifically, they are preferably accumulated in the endosperm. In this case, the mite antigen peptide variants of the present invention can be easily taken up by the body when a human eats the rice that has accumulated these mite antigen peptide variants.

DNA capable of expressing in a rice plant the mite antigen peptide variants of the present invention used in the methods of the invention is also included in the present invention. Such DNA can be exemplified by DNA having a structure in which any one of the following DNA is placed under the control of a storage protein promoter.
(a) DNA in which DNA encoding the storage protein signal sequence is added to the 5'-end of DNA encoding a mite antigen peptide variant, and/or DNA encoding the ER-retention signal sequence to the 3'-end thereof;
(b) DNA encoding a polypeptide in which the storage protein signal sequence is added to the N terminus of a mite antigen peptide variant, and/or the ER-retention signal sequence to the C terminus thereof.

The phrase "DNA is placed under the control of a storage protein promoter" indicates that the storage protein promoter and the DNA are linked to enable the expression of the DNA. That is, it means that the promoter and the DNA downstream thereof are linked such that the expression of the DNA downstream of the promoter is induced when the promoter is transcriptionally activated.

Further, another embodiment of the present invention provides a method of inserting a mite antigen peptide variant into a storage protein and expressing/accumulating the epitope as a part of the storage protein.

In a preferred embodiment of this method, first DNA encoding the mite antigen peptide variant is obtained, and then the DNA is inserted into a DNA region encoding a variable region of a plant storage protein to be expressed.

In this method, the insertion site for the mite antigen peptide variant in a storage protein is preferably a variable region of the storage protein. The phrase "gariable region" refers to a region that has a great variety in terms of the type and length of amino acid sequence acquired over the course of evolution. Therefore, since the insertion of foreign peptides does not affect the three-dimensional structure, it is possible to accumulate a foreign peptide as a part of the storage protein. In addition, since the foreign peptide behaves as part of the storage protein, it is possible to accumulate it in the same storage site as that of the peptide-introduced storage protein.

Variable regions used in the present invention include, for example, in the case of rice glutelin, three regions of the acidic subunit (in the case of the glutelin GluB-1 gene, regions of amino acids 140, 210, and 270 to 310 counted from the N terminus) and the C-terminal region of the basic subunit. Accordingly, a foreign peptide to be expressed can be inserted into these regions. It is also possible to insert the mite antigen peptide variants of the present invention, one into each of the above-described respective variable regions. Glutelin belongs to the 11S globulin family (soybean glycinin and oats globulin are members), and thus a foreign peptide can be inserted into the variable regions exemplified above of other proteins belonging to this family. However, in the case of rice globulin (as distinguished from oats globulin), it is possible to insert the epitope into the variable region located about 110 amino acids from the N terminus.

In this method, insertion of DNA encoding the epitope of the present invention into a DNA region encoding the variable region of the storage protein can be readily performed by one skilled in the art using standard genetic engineering techniques.

DNA encoding a polypeptide having a structure in which the mite antigen peptide variants used in the above-described method are inserted into the storage protein is also included in the present invention. Such DNAs include, for example, (c) DNA encoding a polypeptide having a structure in which a mite antigen peptide variant is inserted into the amino acid sequence (preferably the variable region) of the storage protein under the control of the storage protein promoter. Usually, the promoter originally present upstream of the storage protein gene can be used as the promoter.

Specific examples of the mite antigen peptide variants of the present invention which can be expressed in the rice seed endosperm include peptides encoded by DNAs comprising the nucleotide sequence of any one of:
(a) a DNA comprising the nucleotide sequence of SEQ ID NO: 9;
(b) a DNA which hybridizes under stringent conditions to a DNA comprising the nucleotide sequence of SEQ ID NO: 9;
(c) a DNA encoding a protein comprising the amino acid sequence of SEQ ID NO: 10; and
(d) a DNA encoding a protein which comprises an amino acid sequence with a substitution, deletion, insertion, and/or addition of one or more amino acids in the amino acid sequence of SEQ ID NO: 10.

The present invention also provides a vector comprising DNA of the present invention and a host cell harboring a DNA or vector of the present invention. Vectors of the present invention are not particularly limited, as long as they are capable of stably retaining DNA of the invention. Vectors of the present invention can be prepared by one skilled in the art by appropriately considering the type of plants in which the DNAs are to be expressed, and cloning the above-described DNAs into known various vectors. Insertion of DNA of the present invention into known vectors can be performed by a standard method, for example, by ligase reaction using a restriction enzyme site (Current protocols in Molecular Biology edit. Ausubel et al. (1987) Publish. John Wiley & Sons. Section 11.4-11.11).

Examples of vectors of the present invention include the vector pGluBsig7CrpKDEL. The vector of the present invention can be used in the epitope accumulation method of the present invention, and is therefore useful as a vector for preparing mite antigen peptide variant accumulated plants.

Host cells into which the vector of the present invention is to be introduced are not particularly limited, and various known cells may be used according to the objectives. The phrase "gost cells" as used herein includes rice plant cells of all forms capable of regenerating a plant body. Host cells include, for example, suspended culture cells, protoplasts, shoot primordia, multiple shoots, hairy roots, and calluses, but are not limited to them. When the purpose is to preserve or reproduce the vector of the present invention and the like, host cells of the present invention need not necessarily be plant-derived cells, and may be, for example, *E. coli,* yeast, or animal cells.

Introduction of vectors into host cells can be performed by known methods, such as the calcium phosphate precipitation method, the electroporation method (Current protocols in Molecular Biology edit. Ausubel et al. (1987) Publish. John Wiley & Sons. Section 9.1-9.9), lipofectamine method (GIBCO-BRL), and the microinjection method.

The present invention also relates to a method for accumulating a mite antigen peptide variant in a rice plant comprising the step of introducing a DNA or vector of the present invention into a rice plant, as well as a method for producing a plant comprising a mite antigen peptide variant accumulated therein comprising the step of introducing a DNA or vector of the present invention into a rice plant. A preferred embodiment of the present invention provides a method for producing a transgenic rice plant comprising a mite antigen peptide variant accumulated therein and a method for producing rice comprising a mite antigen peptide variant accumulated therein using techniques of the present invention.

When producing a transformant rice plant comprising a mite antigen peptide variant accumulated therein using DNA of the present invention, for example, DNA of the present invention may be inserted into an appropriate vector, which may then be introduced into a rice plant cell, and the transformant rice plant thus obtained may be cultivated (regenerated). Cultivation (regeneration) of a plant body can be carried out by a method known to one skilled in the art (see Toki et al. (1995) Plant Physiol. 100: 1503-1507). As for rice, several techniques of producing transformant plant body have been established, such as the method of introducing a gene into protoplast with polyethylene glycol to grow (regenerate) a plant body (Datta, S.K. (1995) In Gene Transfer To Plants (Potrykus, I. and Spangenberg Eds.) pp. 66-74); the method of introducing a gene into protoplast by electric pulse to grow (regenerate) a plant body (Toki et al. (1995) Plant Physiol. 100, 1503-1507); the method of directly introducing a gene into cells by the particle-gun method to grow (regenerate) a plant body (Christou et al. (1991) Bio/technology, 9: 957-962); and the method of introducing a gene via *Agrobacterium* to grow (regenerate) a plant body (Hiei et al. (1994) Plant J. 6: 271-282). These techniques are broadly used in the technical field of the present invention. In the present invention, these methods can be appropriately used. When the *Agrobacterium* method is used, for example, the method of Nagel *et al.* is preferably employed (Microbiol. Lett., 1990, 67, 325). According to this method, a vector is introduced into *Agrobacterium* cells, and subsequently the transformed *Agrobacterium* cells are introduced into rice plant cells by known methods, such as the leaf disc method.

Further, plants to be transduced with the DNA or vectors of the present invention may be explants. Cultured cells may be prepared from these plants, and then the DNA or vector can be introduced into the cultured cells. Examples of "plant cells" of the present invention include plant cells of leaf, root, stem, flower, and scutellum in the seed, callus, suspended culture cells, etc.

Further, in order to efficiently select plant cells transformed by introduction of a DNA or nucleic acid of the present invention, a DNA or vector of the present invention preferably contains an appropriate selective marker gene, or is introduced into plant cells together with a plasmid vector containing such a selective marker gene. Examples of selective marker genes used for this purpose are the hygromycin phosphotransferase gene resistant to the antibiotic hygromycin; the neomycin phosphotransferase gene resistant to kanamycin or gentamycin; the acetyl transferase gene resistant to the herbicide phosphinothricin, and the like.

Plant cells transduced with a recombinant vector may be plated and cultured on a known selective medium containing an appropriate selective drug according to the type of the selective marker gene introduced. Through this method, cultures of transformed plant cells can be obtained.

A plant body regenerated from the transformant cells is then cultured in the conditioned medium for acclimatization. Subsequently, the acclimatized regenerated plant body is cultivated under normal cultivation conditions to obtain the plant body which may ripen, bear fruits, and yield seeds.

The presence of DNA of the present invention introduced into the transformant plant body thus cultivated (regenerated) can be confirmed by known PCR method and Southern hybridization method. In this case, extraction of DNA from the transformant plant body can be performed according to the known method of J. Sambrook et al. (Molecular Cloning, 2nd ed., Cold Spring Harbor Laboratory Press, 1989).

The present invention also provides transgenic (transformed) rice plants that have accumulated mite antigen peptide variants, which are produced by the methods of the present invention, and cells derived from these rice plants. Rice plants in which a mite antigen peptide variant has accumulated in the seeds by the methods of the present invention are useful, for example, as mite allergy-alleviating crops.

Once a transformant rice plant having a mite antigen peptide variant of the present invention accumulated therein is obtained, it is possible to produce progenies from the plant body by sexual or asexual reproduction. It is also possible to obtain breeding materials (such as seeds, cuttings, stumps, calluses, and protoplasts) from the plant body and progeny or clone thereof, and mass-produce the plant body based on these materials. The present invention includes progenies or clones of the transgenic rice plant in which a mite antigen peptide variant produced by the method of the present invention is accumulated, cells, breeding materials as well as seeds derived from the transgenic rice plant or progenies and clones thereof. Seeds of the present invention are expected to have thermostability. A preferred embodiment of the present invention includes, for example, rice (rice plant) in which a mite antigen peptide variant is accumulated in endosperm.

Further, the present invention provides food compositions and food/drink products having an effect of preventing, treating, or alleviating allergic diseases. Food compositions or food/drink products of the present invention are composed of parts (*e.g.*, seed (rice)) in which the mite antigen peptide variants produced by the method of the present invention are accumulated, or extracts containing the mite antigen peptides extracted from these parts, or processed products thereof. More specifically, they are food compositions or food/drink products for the treatment or prevention of allergic diseases, which contain seeds (rice) that have accumulated the mite antigen peptide variants obtained by the method of the present invention, or ingredients extracted from them. The "gomposition" in the context of the present invention may be a food composed solely of the seeds (rice) of the present invention, and it is not necessary to supplement the seeds (rice) with multiple types of substances.

Furthermore, as food compositions food/drink products of the present invention, of the rice that has accumulated the mite antigen peptide variants of the present invention in the endosperm, several types of rice that have accumulated variants from different antigen proteins (for example, Der p1, Der p2, Der f1, or Der f2) or variants having different T cell epitopes derived from a same antigen protein may be mixed.

It is also possible to process the food compositions or food/drink products of the present invention by cookery, such as heating. They can also be processed to be used as a health-promoting food, functional food, specific health-preservation food, nutritional supplement food, and the like by mixing them with food hygienically acceptable additives. The food composition is appropriately provided with additives, such as a stabilizer, preservative, colorant, food flavor, and/or vitamins, then mixed, and can be processed by a standard method into forms suitable for the composition, such as a tablet, particle, granule, powder, capsule, liquid, cream, and drink.

A preferred embodiment of the present invention is a food/drink product containing the rice that has accumulated mite antigen peptide variants, which has an effect of preventing, treating, or alleviating allergic diseases (*e.g.*, mite allergy). The food and drink products include processed products made from the rice of the present invention (ingredient) and are for example, rice cakes (dumpling, sliced rice cake, polished rice flour, glutinous rice flour), rice crackers, rice noodles, refined sake, brown rice tea, rice bran, noodles, etc.

The food compositions or food/drink products of the present invention preferably include an indication that they are intended to be used for preventing, treating, or alleviating allergic diseases (*e.g.*, mite allergy).

The present invention also includes pharmaceutical compositions for the treatment or prevention of allergic diseases containing the parts (such as seeds (rice)) that have accumulated the mite antigen peptide variants produced by the method of the present invention as an effective ingredient.

The subjects to be administered with the compositions of the present invention are mammals. A preferred mammal is human. Preferred administration methods include, for example, oral administration. The administration methods can be selected properly according to the patient'fs age and codition. Alternatively, after purification, mite antigen peptides of the present invention can be administered by intravenous injection such as drip infusion, intramuscular injection, intraperitoneal injection, or subcutaneous injection, or as suppositories, enemas, or the like. The compositions of the present invention may contain pharmaceutically acceptable carriers, such as preservatives and stabilizers.

When the parts (seeds (rice), etc) that have accumulated the mite antigen peptide variants generated by the methods of the present invention are used to treat or prevent allergic diseases, or to alleviate their symptoms, the total amount administered (ingested) from the beginning to the end of administration is 1 kg to 30 kg, preferably 1 kg to 15 kg (per 50 kg of body weight). The number of administration (ingestion) varies depending on the administration method and symptoms; for example, the rice is preferably administered at a dose of about 100 to 200 g/day, preferably about 150 g/day over an administration period of one month or longer, preferably three to twelve months, and more preferably about three to six months. The administration intervals in the administration period can be appropriately adjusted according to symptoms and such.

Herein, "allergic disease" is a collective name for diseases involving allergic response. Furthermore, allergic diseases can be typically divided into disease groups showing type I to IV allergic reactions. Allergic diseases in the present invention are not particularly limited; however, they are preferably type I allergies. Allergic diseases in the present invention include not only acute allergic diseases in which an immediate allergic response is triggered against an allergen, but also chronic allergic diseases.

Furthermore, as used herein, "to treat an allergic disease"h means that an allergy symptom is eliminated or alleviated by administering to a subject a food composition, a food/drink product, or a pharmaceutical composition of the present invention. Meanwhile, as used herein, "to prevent an allergic disease"h means to prevent contacting an allergic disease caused by a particular allergen, or to prevent developing symptoms of the allergic disease.

Preferred allergic diseases in the present invention include mite allergy. Allergic disease symptoms include, for example, respiratory diseases such as bronchial asthma, and skin diseases such as atopic dermatitis. Even when such symptoms are mild or not manifested, the development of an allergic disease can assessed based on an increase in the level of antigen-specific IgE antibodies. Therapeutic application of the above-described methods of the present invention which use allergen-specific epitopes corresponding to the diseases is expected in the so-called tailor-made medicine.

All prior-art documents cited herein are incorporated by reference into this description.

### Examples

Hereinbelow, the present invention is specifically described with reference to the Examples; however, it should not be construed as being limited thereto.

### [Example 1] Expression and accumulation of Der p1 peptide in rice seeds

The gene (Der p1 45-145) encoding a part (amino acids: Ser⁴⁵ to Asp¹⁴⁵) of Der p1 protein was synthesized in accordance with the frequency of codons used in rice seeds. The nucleotide and amino acid sequences are shown in Fig. 1, and also in SEQ ID NOs: 9 and 10. An *Nco*I site and a *Sac*I site were added to the 5'f and ' f ends, respectively, for subsequent cloning. Furthermore, the sequence encoding KDEL (Lys-Asp-Glu-Leu), a signal for the endoplasmic reticulum was attached to the C terminus of the amino acid sequence, and a stop codon was added immediately after the signal. This gene fragment was inserted between the GluB-1 promoter and terminator, and subcloned into pGPTV, a plant transformation vector, to prepare a construct for plant transformation (Fig. 2A).

Rice seed-derived calluses were infected with the construct using the Agrobacterium EHA105 strain to create transformed rice plants. Proteins were extracted from seeds of about 40 transformants thus obtained, and transformants with maximal accumulation of Der p1 peptide were selected. Selection was carried out by quantitation through detection and color development with an anti-Der p1 antibody and comparison of the amount accumulated in rice seeds. The result showed that Der p1 peptide was most accumulated in plant No. 28 and the accumulated amount was 89.3 µg per grain of seed (Fig. 2B). Der p1 peptide in the seeds of this line was further analyzed by SDS-PAGE and Western blotting. The SDS-PAGE analysis confirmed in the lane for Der p1-expressing rice, a band at the position of about 12 kDa molecular weight, which was not detected in the lane for non-transformed Kita-ake (arrow in the left panel of Fig. 2C). This molecular weight nearly matched the molecular weight (12.0 kDa) estimated from the amino acid sequence of the designed Der p1 peptide. Thus, the Der p1 peptide was revealed to be accumulated in rice seeds at a level that could be visualized by CBB staining. Furthermore, Western blot analysis was carried out using an anti-Der p1 antibody.
As a result, the anti-Der p1 antibody detected a single signal at the same position as that of the band visualized by CBB in the SDS-PAGE analysis, confirming that the band was the product of the introduced Der p1 gene (arrow in the right panel of Fig. 2C). Meanwhile, no signal was detected in the lane in which proteins from non-transformed Kita-ake were electrophoresed.

Southern hybridization analysis was carried out to examine the level of integration by the gene of interest in the genome of transformant No. 28 obtained by Agrobacterium-mediated rice plant transformation. Genomic DNAs were purified from transformant No. 28 and non-transformants, digested with the restriction enzymes *Eco*RI and *Hin*dIII, and DNA fragments were separated by electrophoresis using a 0.8% agarose gel. After electrophoresis, the DNA fragments were transferred onto Hybond-N⁺ membrane by the alkaline transfer method. Der p1-specific signals were detected on the membrane by using ³²P-labeled Der p1 (45-145) DNA probe. As a result, signals were detected at positions of about 7 kbp, 9 kbp, and 1.2 kbp in the lane in which the genomic DNA of transformant No. 28 was electrophoresed following *Eco*RI digestion, while a signal was detected at positions of about 7 kbp and 9 kbp in the lane in which the genomic DNA of transformant No. 28 was electrophoresed following *Hin*dIII digestion. Meanwhile, no signal was detected in the lane in which the genomic DNA of non-transformed Kita-ake digested in the same way was electrophoresed (Fig. 2D).

### [Example 2] Effect of Der p1 rice orally administered to mice

Each of the control rice and Der p1 transformant rice (hereinafter also referred to as Der p1 rice) obtained in Example 1 was ground in a food processor, combined with commercially available powdered mouse feed at a ratio of 1:1, and orally administered to mice by free uptake for seven days. Then, for immunization, a suspension containing 5 µg of Der p1 protein and 0.4 mg of Alum was intraperitoneally administered to the subject mice four times at one week intervals. Sera were collected seven days after the final immunization, and the antibody titers of Der p1-specific IgG and IgE were determined by ELISA. The schedule of administration and subsequent immunization is shown in Fig. 3A. According to the result, Der p1-specific IgG and IgE were detected at high levels in mice orally administered with the control rice as compared to in the sera of naive mice, while Der p1-specific IgG and IgE were detected only at low levels in sera of mice orally administered with Der p1 rice (Figs. 3B and 3C). Furthermore, the total amount of IgE in the sera obtained from mice of each experimental group were determined, and the result showed that the concentration in the blood was significantly reduced in mice administered with Der p1 rice as compared to mice administered with the control rice (Fig. 3D, control rice-administered group: 8.56 ng/ml; Der p1 rice-administered group: 3.42 ng/ml).

### [Example 3] Subclass analysis of Der p1-specific IgG

The subclasses of IgGs in the sera collected from mice of each experimental group were analyzed. The measurement was carried out by the ELISA method, similarly as described above. IgG₁ and IgG_{2b} were efficiently induced by immunization with Der p1 in the sera of mice administered with the control rice, when compared to naive mice. In contrast, induction of antibodies of these IgG subclasses was not detected in the sera of mice administered with Der p1 rice (Figs. 4A and 4C). There was no significant difference in the antibody titer between the IgG₂ₐ and IgG₃ subclasses (Figs. 4B and 4D).

### [Example 4] Antigen-specific T cell proliferation and cytokine release

The spleen was excised from mice orally administered with each of the rice, CD4⁺ T cells were purified, and antigen-specific T cell proliferation assay was carried out. CD4⁺ T cells prepared from mice of the control rice-administered experimental group proliferated well upon stimulation with Der p1 antigen. However, CD4⁺ T cells prepared from mice of the Der p1 rice-administered experimental group hardly proliferated after antigen stimulation (Fig. 5A). Culture supernatants of these cells were collected, and the amount of cytokines released from the cells was measured. The Th2 cytokines IL-4, -5, and -13 were released at high levels from the CD4⁺ T cells of mice administered with the control rice. However, the CD4⁺ T cells of mice administered with Der p1 rice hardly released these Th2 cytokines (Figs. 5B, 5C, and 5D). Meanwhile, INF-γ, a Th1-type cytokine, was only released at a low level in each of the experimental groups, and no significant difference was observed (Fig. 5E).

### [Example 5] Change in the composition of cells in bronchoalveolar lavage fluid

Asthma is a major symptom in mite allergy. Thus, Der p1 rice was assessed for its effect on asthmatic symptom. Mice orally administered with the control rice or Der p1 rice were immunized with Der p1, and 14 days after the final immunization, 10 µg of Der p1 antigen was intranasally administered to each of the mice intraperitoneally anesthetized with Nembutal. Bronchoalveolar lavage fluid (BALF; 1 ml/mouse) was collected 24 hours after antigen challenge, and then Diff Quick staining was performed to determine the number of cells contained in the BALF to assess the difference in the composition of cells induced, and such. The result showed that in mice administered with the control rice, typical symptoms observed in the asthma models and infiltration of macrophages and eosinophils were seen, and the number of cells infiltrated into the lungs and bronchi had also increased. In contrast, as shown in Fig. 5C, infiltration of eosinophils was hardly observed in conjunction with the decrease of the IL-5 production level in mice administered with Der p1 rice, and the number of macrophages or the like induced by antigen stimulation was also drastically reduced (Fig. 6A and 6B).

### [Example 6] Enhancement of airway hypersensitivity by Der p1 rice administration

Next, antigen sensitization and nasal antigen challenge were performed by similar methods as described above to each experimental group, and enhancement of the airway hypersensitivity was examined by measuring the airway resistance. An increase of airway resistance was observed upon antigen challenge as a typical symptom of asthma in mice administered with the control rice. In contrast, such an increase in the airway resistance was not observed in mice administered with Der p1 rice (Fig. 6C).

### [Example 7] Histochemical analysis of lung after administration of Der p1 rice

Next, lung inflammation due to Der p1 rice administration was assessed by preparing and observing cryosections. Mice of each experimental group were intraperitoneally sensitized with Der p1 antigen and transnasally challenged by the methods described above. Lungs were then excised from each mouse and embedded in OCT compound. This was frozen in liquid nitrogen and cryosections with a thickness of 5 µm were prepared. Lung inflammation was observed by staining the obtained cryosections with hematoxylin/eosin (Fig. 7) or PAS (Fig. 8). The result showed that antigen sensitization and nasal challenge induced inflammation in the lung such that a large number of cells such as macrophages and eosinophils which infiltrated into the lung were seen in the experimental group of mice administered with the control rice. In particular, eosinophils have a segmented nucleus and are recognized as cells whose intracellular granules are stained in red by eosin (white arrows in Fig. 7B). Furthermore, epithelial cells in the airway tract periphery were thickened (black arrows in Fig. 7B). Meanwhile, the experimental group of mice administered with Der p1 rice had a comparable condition to that of naive mice that received the challenge, and neither infiltration of cells such as macrophages and eosinophils nor thickening of epithelial cells was observed (Fig. 7C). In specimens of cryosections similarly prepared and PAS stained, inflammation was observed by PAS staining of the lungs of mice administered with the control rice which showed red staining of mucin secreted from goblet cells among the epithelial cells in the airway tract periphery (arrows in Fig. 8B). Meanwhile, although some cells in the group of mice orally administered with Der p1 rice were stained with PAS, the number of stained goblet cells was significantly reduced as compared to that in mice administered with the control rice (Fig. 8C).

### [Example 8] Effect of oral administration of Der f2ΔC rice to mice

Each of the control rice and Der f2 ΔC-expressing rice (hereinafter also referred to as Der f2 ΔC rice; Fig. 11) prepared by a similar method described in Example 1 were ground in a food processor, combined with commercially available powdered mouse feed at a ratio of 1:1, and orally administered to mice by free uptake for one month. Then, for immunization, a suspension containing 0.5 µg of wild type Der f2 protein and 0.4 mg of Alum was intraperitoneally administered to the subject mice four times at one week intervals. Sera were collected seven days after the final immunization, and the antibody titers of wild-type Der f2-specific IgG and IgE were determined by ELISA. The schedule of administration and subsequent immunization is shown in Fig. 12A. According to the result, there was no significant difference in the amounts of wild-type Der f2-specific IgG and IgE produced in the serum between mice orally administered with Der f2 ΔC rice and mice administered with the control rice (Figs. 12B and 12C). Furthermore, the total amount of IgE in the sera obtained from mice of each experimental group was measured, and the result showed that a slight decrease was observed in mice administered with Der f2 ΔC rice as compared to mice administered with the control rice although a significant difference was not observed (Fig. 12D).

### [Example 9] Effect of oral administration of Der f2 rice (mixture of C8/119 rice and C21/27, C73/78 rice) on mice

The control rice as well as a rice mixture (hereinafter also referred to as Der f2 rice) of Der f2 (C8/119) transformant rice and Der f2 (C21/27, C73/78) transformant rice (Fig. 11) prepared by a similar method described in Example 1 and mixed in equal amounts were ground in a food processor, combined with commercially available powdered mouse feed at a ratio of 1:1, and orally administered to mice by free uptake for 14 days. Then, for immunization, a suspension containing 0.5 µg of wild-type Der f2 protein and 0.4 mg of Alum was intraperitoneally administered to these subject mice four times at one week intervals. Sera were collected seven days after the final immunization, and the antibody titers of wild-type Der f2-specific IgG and IgE were determined by ELISA. The schedule of administration and subsequent immunization is shown in Fig. 13A. According to the result, wild-type Der f2-specific IgG and IgE were detected at high levels in mice orally administered with the control rice as compared to the levels in the sera of naive mice, while wild-type Der f2-specific IgG and IgE were detected only at low levels in sera obtained from mice orally administered with Der f2 rice (Figs. 13B and 13C). Furthermore, the total amount of IgE in the sera obtained from mice of each experimental group was determined, and the result showed that the concentration in the blood was significantly reduced in mice administered with wild-type Der f2 rice as compared to the concentration in mice administered with the control rice (Fig. 13D).

### [Example 10] Expression and accumulation of Der p1 (full-length) protein in rice seeds

The gene encoding the full-length Der p1 protein was synthesized in accordance with the frequency of codons used in rice seeds. The nucleotide and amino acid sequences are shown in SEQ ID NOs: 9 and 2, respectively (Fig. 16). An *Nco*I site and a *Sac*I site were added to the 5'f and ' f ends, respectively, for subsequent cloning. Furthermore a sequence encoding KDEL (Lys-Asp-Glu-Leu), a signal for the endoplasmic reticulum, was attached to the C terminus of the amino acid sequence, and a stop codon was added immediately after the signal. In addition, Cys at amino acid position 34 was substituted with Ala to eliminate the cysteine protease activity of Der p1 protein (Ψ in Fig. 14A). This gene fragment was inserted between the GluB-1 promoter and terminator, and this was subcloned into pGPTV, a plant transformation vector, to prepare a construct for plant transformation (Fig. 14A).

Rice seed-derived calluses were infected with the construct using the Agrobacterium EHA105 strain to create transformed rice plants. Proteins were extracted from seeds of about 30 transformants thus obtained, and transformants with maximal accumulation of Der p1 protein were selected. Selection was carried out by quantitation through detection and color development with anti-Der p1 antibody and comparison of the amount accumulated in rice seeds. The result showed that Der p1 peptide was most accumulated in plant No. 17 and the accumulated amount was 58 µg per grain of seed (Fig, 14B), which accounts for about 4% of the total seed protein when converted. Der p1 peptide in the seeds of this line was further analyzed by SDS-PAGE and Western blotting. The SDS-PAGE analysis confirmed in the lanes (3 and 4) for Der p1-expressing rice, bands at positions of about 25 to 27 kDa molecular weights, which were not detected in the lanes (1 and 2) for non-transformed Kita-ake (left panel of Fig. 14C). Furthermore, Western blot analysis was carried out using an anti-Der p1 antibody. As a result, the anti-Der p1 antibody detected signals at 25 kDa and 27 kDa positions, the same positions as the bands visualized by CBB in the SDS-PAGE analysis, confirming that these bands were the product of the introduced Der p1 gene (right panel of Fig. 14C). The band at the 25 kDa MW position nearly matched 25 kDa, which is the molecular weight estimated from the amino acid sequence of the designed Der p1 peptide. Since Der p1 is N-glycosylated at Asn of position 52, the band found at about 27 kDa may be the N-glycosylated peptide that had accumulated in the seeds in a sugar chain-attached form. Meanwhile, no signal was detected in the lane in which proteins of the non-transformant Kita-ake were electrophoresed. Furthermore, intracellular localization of Der p1 protein was examined by electron microscopy using a gold colloid-linked Der p1-specific antibody. The result revealed that the Der p1 protein expressed and accumulated in rice seeds was accumulated in endoplasmic reticulum-derived protein body I (Fig. 14D).

### [Example 11] Analysis of sugar chain of Der p1 protein accumulated in rice seeds and reactivity with IgE

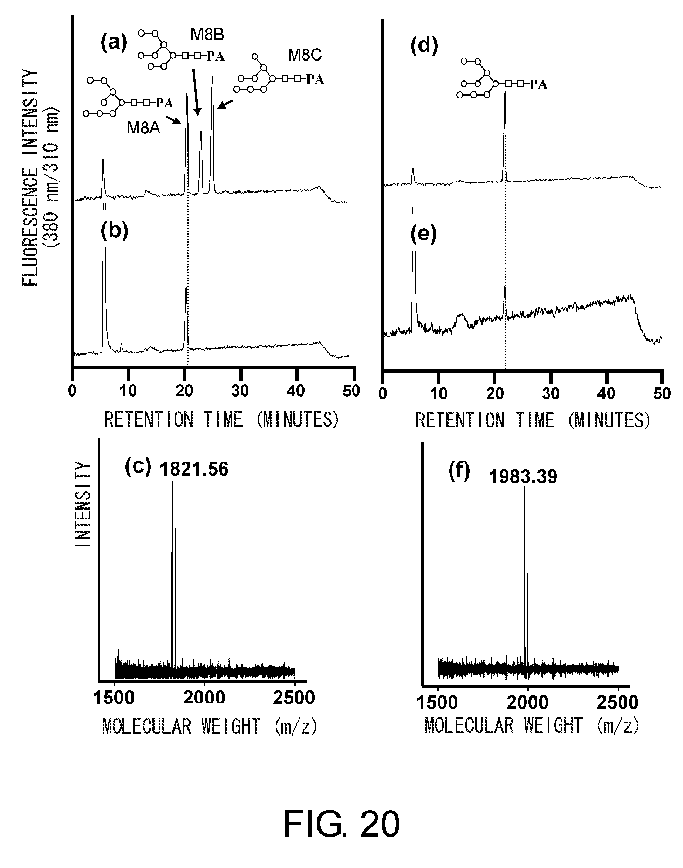

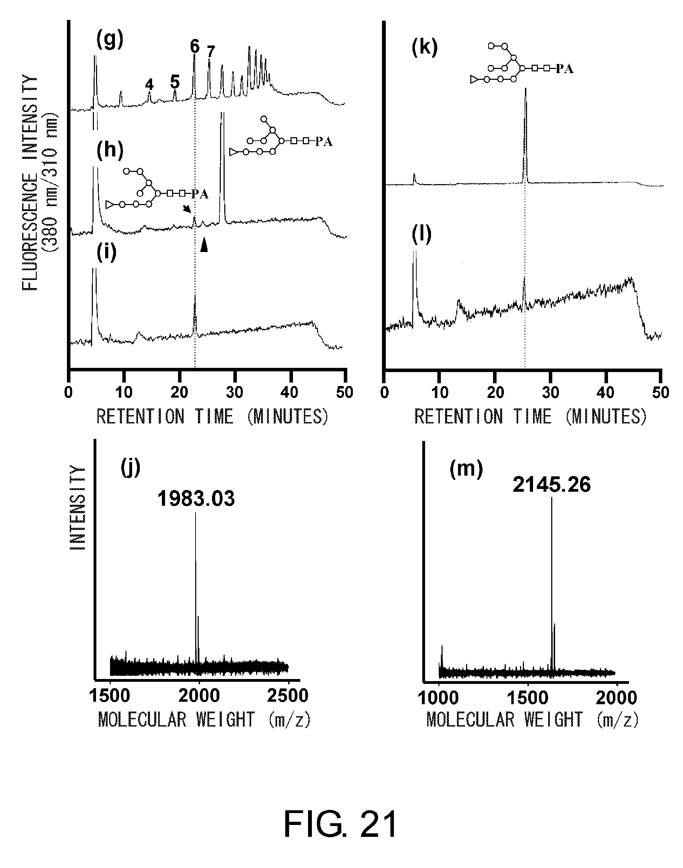

The structure of the sugar chain added to the Der p1 protein accumulated in rice seeds was assessed. First, treatment with EndoH, an enzyme that cleaves mannose type sugar chains, was carried out. As a result, SDS-PAGE and Western blot analyses revealed that the band detected at about 27 kDa disappeared after enzyme treatment. Further, the structure of the sugar chain was analyzed in detail using MALDI-TOF MS. The result showed that the sugar chains attached to Der p1 were mostly Man₈GlcNac₂ (Fig. 20, a, b, and c), Man₉GlcNac₂ (Fig. 20, d, e, and f), Glu₁Man₈GlcNac₂ (Fig. 21, g, h, i, and j), and Glu₁Man₉GlcNac₂ (Fig. 21, k, l, and m). These sugar chains accounted for 91.4% of total, and these sugar chains were the same as the attached sugar chains in mammalian cells.

Der p1 protein accumulated in rice seeds with added sugar chains and Der p1 protein produced in *Escherichia coli* without sugar chain were examined for their reactivity with IgE in the sera of mite allergy patients. Analysis was carried out by Western blotting, and the intensity of color development was quantified. The result revealed that in the sera of most patients, Der p1 protein expressed in rice seeds with added sugar chains (lane 2 in Fig. 15B) was less responsive to IgE as compared to Der p1 protein with no sugar chain (lane 1 in Fig. 15B). Furthermore, the quantitation result on the reactivity of IgE demonstrated that the reactivity was reduced to about 30% for those with added sugar chains as compared to those with no sugar chain (Fig. 15C).

### Industrial Applicability

With the present invention, mite allergen peptide variants having an effect of alleviating (treating) mite allergic diseases have been successfully produced in rice seeds.

Compared to conventional production of peptide proteins by culture of *E. coli* and the like, mite antigen peptide variants can be produced inexpensively by the present invention. Specifically, in the case of rice plant, 100 to 200 seeds can be generated from a single seed. Furthermore, since they can be produced in seeds, they may be orally ingested as is without purification. Peptides produced in seeds are very stable, so that even when seeds are left at room temperature, they are not degraded and their activity is not lost for one year or longer. In addition, the amount of production can be readily controlled as compared to tank culture. The amount of production can be controlled by the number of seeds to be seeded. Furthermore, no special facility is required, only a cultivation field is needed.

In addition, the oral intake through daily diet can save on medical expenses and expenses required for conventional administrations such as subcutaneous injection, and mite antigen peptides can be administered at a lower cost. By using the rice seed production system with these advantages, generation of new businesses is also expected, where pharmaceutically useful substances such as vaccines against allergic diseases and peptides which alleviate lifestyle-related illnesses are produced and supplied at a lower cost.

## Claims

1. A DNA construct having a structure in which the DNA of any one of (a) to (d) below is placed under the control of a seed storage protein promoter of a rice plant:
(a) a DNA encoding a peptide derived from a mite antigen protein, wherein the DNA comprises a DNA encoding a storage protein signal sequence added at the 5'f end and b or a DNA encoding an endoplasmic reticulum retention signal sequence added at the 3' end, and wherein the peptide comprises a plurality of T cell epitopes;
(b) a DNA encoding a peptide derived from a mite antigen protein, wherein the peptide comprises a storage protein signal sequence added at the N terminus and b or an endoplasmic reticulum retention signal sequence added at the C terminus, and wherein the peptide comprises a plurality of T cell epitopes;
(c) a DNA encoding a mite antigen protein, wherein the DNA comprises a DNA encoding a storage protein signal sequence added at the 5'f end and b or a DNAencod ng an endopl as ṁc reticulum retention signal sequence added at the 3' end, and wherein the mite antigen protein is modified to not form a conformation to be recognized as an antigen; and
(d) a DNA encoding a mite antigen protein, wherein the protein comprises a storage protein signal sequence added at the N terminus and o or an endoplasmic reticulum retention signal sequence added at the C terminus, and wherein the protein is modified to not form a conformation to be recognized as an antigen.

2. The DNA construct of claim 1, wherein the mite antigen protein is a type 1 or type 2 antigen protein.

3. The DNA construct of claim 1, wherein the mite antigen protein comprises the amino acid sequence of:
(i) an amino acid sequence of any one of SEQ ID NO: 2, 4, 6, and 8; or
(ii) a DNA encoding a protein which comprises an amino acid sequence with a substitution, deletion, insertion, and o or addition of one or more amino acids in the amino acid sequence of any one of SEQ ID NO: 2, 4, 6, and 8.

4. The DNA construct of claim 1, wherein the peptide that comprises a plurality of T cell epitopes comprises the region of any one of:
(i) two or more regions selected from positions 45 to 67, positions 94 to 104, and positions 117 to 143 in the amino acid sequence of SEQ ID NO: 2;
(ii) two or more regions selected from positions 21 to 49, positions 71 to 100, positions 93 to 108, positions 110 to 131, and positions 197 to 212 in the amino acid sequence of SEQ ID NO: 2;
(iii) two or more regions selected from positions 11 to 35, positions 87 to 104, and positions 105 to 129 in the amino acid sequence of SEQ ID NO: 4; and
(iv) two or more regions selected from positions 35 to 50, positions 35 to 60, and positions 87 to 104 in the amino acid sequence of SEQ ID NO: 4.

5. The DNA construct of claim 1, wherein the peptide comprising a plurality of T cell epitopes is a partial peptide which has been modified so that the peptide does not form a conformation to be recognized as an antigen, and wherein the peptide is:
(i) a peptide which comprises an amino acid sequence comprising the region of positions 45 to 144 in the amino acid sequence of SEQ ID NO: 2; or
(ii) a peptide which comprises an amino acid sequence comprising the region of positions 1 to 144 in the amino acid sequence of SEQ ID NO: 6.

6. The DNA construct of claim 1, wherein the peptide comprising a plurality of T cell epitopes is a peptide which has been modified to not have a cysteine protease activity.

7. The DNA construct of claim 6, wherein the cysteine residue (Cys) at position 34 in the amino acid sequence of SEQ ID NO: 2 is substituted to an alanine residue (Ala).

8. The DNA construct of claim 1, wherein the mite antigen protein modified to not form a conformation to be recognized as an antigen is modified to not have an IgE-inducing active site.

9. The DNA construct of claim 8, wherein the mite antigen protein comprises an amino acid sequence in which any one or more of the cysteine residues (Cys) at positions 8, 21, 27, 73, 78, and 119 in the amino acid sequence of SEQ ID NO: 4 or 8 are modified to a serine residue (Ser).

10. The DNA construct of claim 9, wherein the mite antigen protein is a partial peptide in which a cysteine residue in a partial peptide comprising the region of positions 11 to 129 in SEQ ID NO: 4 or the region of positions 11 to 129 in SEQ ID NO: 8 has been modified.

11. The DNA construct of claim 1, wherein the DNA encoding a mite antigen protein comprises a nucleotide sequence whose codon has been altered to enable its expression in rice endosperm.

12. The DNA construct of claim 1 which comprises the nucleotide sequence of any one of:
(a) a DNA comprising the nucleotide sequence of SEQ ID NO: 9;
(b) a DNA which hybridizes under stringent conditions to a DNA comprising the nucleotide sequence of SEQ ID NO: 9;
(c) a DNA encoding a protein comprising the amino acid sequence of SEQ ID NO: 10; and
(d) a DNA encoding a protein comprising an amino acid sequence with a substitution, deletion, insertion, and o or addition of one or more amino acids in the amino acid sequence of SEQ ID NO: 10.

13. A polypeptide encoded by the DNA construct of any one of claims 1 to 12.

14. The polypeptide of claim 13, which has been added with a sugar chain and enclosed in protein body I.

15. A vector carrying the DNA construct of any one of claims 1 to 12.

16. A transformed rice plant cell harboring the DNA construct of any one of claims 1 to 12, or the vector of claim 15.

17. A transformed rice plant comprising the transformed rice plant cell of claim 16, and accumulating a peptide comprising a plurality of T cell epitopes or a peptide which has been modified to not form a conformation to be recognized as an antigen.

18. The transformed rice plant of claim 16, wherein the peptide is accumulated in a seed of the rice plant.

19. A transformed rice plant, which is a progeny or clone of the transformed rice plant of claim 17.

20. A breeding material of the transformed rice plant of claim 17 or 18.

21. A seed of the transformed rice plant of claim 15 or 18.

22. A food composition for treating or preventing an allergic disease wherein the antigen is a mite, which comprises as an active ingredient the transformed rice plant seed of claim 21.

23. The food composition of claim 22, wherein the allergic disease is type I allergy.

24. A pharmaceutical composition for treating or preventing an allergic disease wherein the antigen is a mite, which comprises as an active ingredient the transformed rice plant seed of claim 21.

25. The pharmaceutical composition of claim 24, wherein the allergic disease is type I allergy.

26. The pharmaceutical composition of claim 24 or 25, which is for oral administration.

27. A method for accumulating in a rice plant a mite antigen protein-derived peptide comprising a plurality of T cell epitopes, wherein the method comprises the steps of:
(a) obtaining a DNA encoding a peptide comprising a plurality of T cell epitopes;
(b) adding a DNA encoding a storage protein signal sequence to the 5'f end and o or a DNA encoding an endoplasmic reticulum retention signal sequence to the 3'f end of the DNA obtained in (a); and
(c) expressing the DNA of (b) in a plant under the control of a storage protein promoter.

28. A method for accumulating in a rice plant a mite antigen protein-derived peptide that has been modified to not form the conformation to be recognized as an antigen, wherein the method comprises the steps of:
(a) obtaining a DNA encoding a mite antigen protein;
(b) modifying the DNA obtained in (a) so that the peptide does not form a conformation to be recognized as an antigen;
(c) adding a DNA encoding a storage protein signal sequence to the 5'f end and o or a DNA encoding an endoplasmic reticulum retention signal sequence to the 3'f end of the DNA obtained in (b); and
(d) expressing the DNA of (c) in a plant under the control of a storage protein promoter.

29. The method of claim 27 or 28, wherein the peptide is accumulated in a seed of the rice plant.

30. A transformed rice plant in which a mite antigen protein-derived peptide comprising a plurality of T cell epitopes and modified to not form a conformation to be recognized as an antigen has been accumulated, wherein the rice plant is produced by the method of any one of claims 27 to 29.

31. A method for treating or preventing an allergic disease, wherein the method comprises the step of orally administering the transformed rice plant seed of claim 21 to a subject.

32. A method for inducing immunological tolerance in a patient having an allergic disease, wherein the method comprises the step of orally administering the transformed rice plant seed of claim 21 to the subject.


SEQUENCE LISTING

<110>  NATIONAL INSTITUTE OF AGROBIOLOGICAL SCIENCES

<120>  MITE ANTIGENIC RICE

<130>  MOA-A0702P

<150> JP 2007-266864
<151> 2007-10-12

<160>  22

<170>  PatentIn version 3.4

<210>  1
<211>  669
<212>  DNA
<213>  Dermatophagoides pteronyssinus

<400>  1
actaacgcct gcagtatcaa tggaaatgct ccagctgaaa tcgatttgcg acaaatgcga      60

actgtcactc ccattcgtat gcaaggaggc tgtggttcat gttgggcttt ctctggtgtt     120

gccgcaactg aatcagctta tttggcttac cgtaatcaat cattggatct tgctgaacaa     180

gaattagtcg attgtgcttc ccaacacggt tgtcatggtg ataccattcc acgtggtatt     240

gaatacatcc aacataatgg tgtcgtccaa gaaagctact atcgatacgt tgcacgagaa     300

caatcatgcc gacgaccaaa tgcacaacgt ttcggtatct caaactattg ccaaatttac     360

ccaccaaatg caaacaaaat tcgtgaagct ttggctcaaa cccacagcgc tattgccgtc     420

attattggca tcaaagattt agacgcattc cgtcattatg atggccgaac aatcattcaa     480

cgcgataatg gttaccaacc aaactatcac gctgtcaaca ttgttggcta cagtaacgca     540

caaggtgtcg attattggat cgtacgaaac agttgggata ccaattgggg tgataatggt     600

tacggttatt ttgctgccaa catcgatttg atgatgattg aagaatatcc atatgttgtc     660

attctctaa                                                             669


<210>  2
<211>  222
<212>  PRT
<213>  Dermatophagoides pteronyssinus

<400>  2

Thr Asn Ala Cys Ser Ile Asn Gly Asn Ala Pro Ala Glu Ile Asp Leu
1               5                   10                  15


Arg Gln Met Arg Thr Val Thr Pro Ile Arg Met Gln Gly Gly Cys Gly
            20                  25                  30


Ser Cys Trp Ala Phe Ser Gly Val Ala Ala Thr Glu Ser Ala Tyr Leu
        35                  40                  45


Ala Tyr Arg Asn Gln Ser Leu Asp Leu Ala Glu Gln Glu Leu Val Asp
    50                  55                  60

Cys Ala Ser Gln His Gly Cys His Gly Asp Thr Ile Pro Arg Gly Ile
65                  70                  75                  80

Glu Tyr Ile Gln His Asn Gly Val Val Gln Glu Ser Tyr Tyr Arg Tyr
                85                  90                  95

Val Ala Arg Glu Gln Ser Cys Arg Arg Pro Asn Ala Gln Arg Phe Gly
            100                 105                 110

Ile Ser Asn Tyr Cys Gln Ile Tyr Pro Pro Asn Ala Asn Lys Ile Arg
        115                 120                 125

Glu Ala Leu Ala Gln Thr His Ser Ala Ile Ala Val Ile Ile Gly Ile
    130                 135                 140

Lys Asp Leu Asp Ala Phe Arg His Tyr Asp Gly Arg Thr Ile Ile Gln
145                 150                 155                 160

Arg Asp Asn Gly Tyr Gln Pro Asn Tyr His Ala Val Asn Ile Val Gly
                165                 170                 175

Tyr Ser Asn Ala Gln Gly Val Asp Tyr Trp Ile Val Arg Asn Ser Trp
            180                 185                 190

Asp Thr Asn Trp Gly Asp Asn Gly Tyr Gly Tyr Phe Ala Ala Asn Ile
        195                 200                 205

Asp Leu Met Met Ile Glu Glu Tyr Pro Tyr Val Val Ile Leu
    210                 215                 220

<210>  3
<211>  390
<212>  DNA
<213>  Dermatophagoides pteronyssinus

<400>  3
gatcaagtcg atgtcaaaga ttgtgccaat catgaaatca aaaaagtttt ggtaccagga      60

tgccatggtt cagaaccatg tatcattcat cgtggtaaac cattccaatt ggaagccgtt     120

ttcgaagcca accaaaacac aaaaacggct aaaattgaaa tcaaagcctc aatcgatggt     180

ttagaagttg atgttcccgg tatcgatcca aatgcatgcc attacatgaa atgcccattg     240

gttaaaggac aacaatatga tattaaatat acatggaatg ttccgaaaat tgcaccaaaa     300

tctgaaaatg ttgtcgtcac tgttaaagtt atgggtgatg atggtgtttt ggcctgtgct     360

attgctactc atgctaaaat ccgcgattaa                                      390

<210>  4
<211>  129
<212>  PRT
<213>  Dermatophagoides pteronyssinus

<400>  4

Asp Gln Val Asp Val Lys Asp Cys Ala Asn His Glu Ile Lys Lys Val

1               5                   10                  15

Leu Val Pro Gly Cys His Gly Ser Glu Pro Cys Ile Ile His Arg Gly
            20                  25                  30

Lys Pro Phe Gln Leu Glu Ala Val Phe Glu Ala Asn Gln Asn Thr Lys
        35                  40                  45

Thr Ala Lys Ile Glu Ile Lys Ala Ser Ile Asp Gly Leu Glu Val Asp
    50                  55                  60

Val Pro Gly Ile Asp Pro Asn Ala Cys His Tyr Met Lys Cys Pro Leu
65                  70                  75                  80

Val Lys Gly Gln Gln Tyr Asp Ile Lys Tyr Thr Trp Asn Val Pro Lys
                85                  90                  95

Ile Ala Pro Lys Ser Glu Asn Val Val Val Thr Val Lys Val Met Gly
            100                 105                 110

Asp Asp Gly Val Leu Ala Cys Ala Ile Ala Thr His Ala Lys Ile Arg
        115                 120                 125

Asp

<210>  5
<211>  672
<212>  DNA
<213>  Dermatophagoides farinae

<400>  5
acaagcgctt gccgtatcaa ttcggttaac gttccatcgg aattggattt acgatcactg      60

cgaactgtca ctccaatccg tatgcaagga ggctgtggtt catgttgggc tttctctggt     120

gtcgccgcaa ctgaatcagc ttatttggcc taccgtaaca cgtctttgga tctttctgaa     180

caggaactcg tcgattgcgc atctcaacac ggatgtcacg gcgatacaat accaagaggc     240

atcgaataca tccaacaaaa tggtgtcgtt gaagaaagaa gctatccata cgttgcacga     300

gaacaacaat gccgacgacc aaattcgcaa cattacggta tctcaaacta ctgccaaatt     360

tatccaccag atgtgaaaca aatccgtgaa gctttgactc aaacacacac agctattgcc     420

gtcattattg gcattaaaga tttgagagct tttcaacatt atgatggacg aacaatcatt     480

caacatgaca atggttatca accaaactat catgccgtca acattgtcgg ttacggaagt     540

acacaaggcg tcgattattg gatcgtacga aacagttggg atactacctg gggtgatagc     600

ggatacggat atttccaagc cggaaacaac ctcatgatga tcgaacaata tccatatgtt     660

gtaatcatgt ga                                                         672

<210>  6
<211>  223
<212>  PRT

<213>  Dermatophagoides farinae

<400>  6

<210>  7
<211>  390
<212>  DNA
<213>  Dermatophagoides farinae

<400>  7
gatcaagtcg atgttaaaga ttgtgccaac aatgaaatca aaaaagtaat ggtcgatggt      60

tgccatggtt ctgatccatg catcatccat cgtggtaaac cattcacttt ggaagcctta     120

ttcgatgcca accaaaacac taaaaccgct aaaattgaaa tcaaagccag cctcgatggt     180

cttgaaattg atgttcccgg tatcgatacc aatgcttgcc attttatgaa atgtccattg     240

gttaaaggtc aacaatatga tgccaaatat acatggaatg tgccgaaaat tgcaccaaaa     300

tctgaaaacg ttgtcgttac agtcaaactt gttggtgata atggtgtttt ggcttgcgct     360

attgctaccc atggtaaaat ccgtgattaa                                      390

<210>  8
<211>  129
<212>  PRT
<213>  Dermatophagoides farinae

<400>  8

Asp Gln Val Asp Val Lys Asp Cys Ala Asn Asn Glu Ile Lys Lys Val
1               5                   10                  15

Met Val Asp Gly Cys His Gly Ser Asp Pro Cys Ile Ile His Arg Gly
            20                  25                  30

Lys Pro Phe Thr Leu Glu Ala Leu Phe Asp Ala Asn Gln Asn Ser Lys
        35                  40                  45

Thr Ala Lys Ile Glu Ile Lys Ala Ser Leu Asp Gly Leu Glu Ile Asp
    50                  55                  60

Val Pro Gly Ile Asp Thr Asn Ala Cys His Phe Val Lys Cys Pro Leu
65                  70                  75                  80

Val Lys Gly Gln Gln Tyr Asp Ile Lys Tyr Thr Trp Asn Val Pro Lys
                85                  90                  95

Ile Ala Pro Lys Ser Glu Asn Val Val Val Thr Val Lys Leu Ile Gly
            100                 105                 110

Asp Asn Gly Val Leu Ala Cys Ala Ile Ala Thr His Gly Lys Ile Arg
        115                 120                 125

Asp

<210>  9
<211>  329
<212>  DNA
<213>  Artificial

<220>
<223>  An artificially synthesized nucleotide sequence

<400>  9
ccatgggatc agcttatttg gcttaccgta atcaatcatt ggatcttgct gagcaggaat      60

tggtagattg tgcttcccaa cacggatgtc atggtgatac cattccacgt ggtattgaat     120

acatccaaca taatggagtt gtacaggaga gctactatcg ctacgttgca cgtgaacaat     180

catgccgccg tccaaatgca caacgtttcg gaatctcaaa ctattgccag atttacccac     240

caaatgtaaa caagattcgt gaggctttgg cacaaaccca cagcgctatt gcagttatta     300

ttggcatcaa ggacgagttg taagagctc                                       329

<210>  10
<211>  106
<212>  PRT
<213>  Artificial

<220>
<223>  An artificially synthesized peptide sequence

<400>  10

Met Gly Ser Ala Tyr Leu Ala Tyr Arg Asn Gln Ser Leu Asp Leu Ala
1               5                   10                  15

Glu Gln Glu Leu Val Asp Cys Ala Ser Gln His Gly Cys His Gly Asp
            20                  25                  30

Thr Ile Pro Arg Gly Ile Glu Tyr Ile Gln His Asn Gly Val Val Gln
        35                  40                  45

Glu Ser Tyr Tyr Arg Tyr Val Ala Arg Glu Gln Ser Cys Arg Arg Pro
    50                  55                  60

Asn Ala Gln Arg Phe Gly Ile Ser Asn Tyr Cys Gln Ile Tyr Pro Pro
65                  70                  75                  80

Asn Val Asn Lys Ile Arg Glu Ala Leu Ala Gln Thr His Ser Ala Ile
                85                  90                  95

Ala Val Ile Ile Gly Ile Lys Asp Glu Leu
            100                 105

<210>  11
<211>  435
<212>  DNA
<213>  Artificial

<220>
<223>  An artificially synthesized nucleotide sequence

<400>  11
actagtgcat gcaggatcaa cagcgttaat gtaccatctg agcttgatct cagaagtttg      60

aggacagtta ctccaatccg tatgcaggga ggctgcggct ctgcttgggc attcagcggc     120

gtagcagcta ccgaatcagc ttatttggct taccgtaata cttcattgga tctttctgag     180

caggaattgg tagattgtgc ttcccaacac ggatgtcatg gtgataccat tccacgtggt     240

attgaataca tccaacaaaa tggagttgta gaggagagaa gctatcccta cgttgcacgt     300

gaacaacaat gccgccgtcc aaattcacaa cattacggaa tctcaaacta ttgccagatt     360

tacccaccag atgtaaagca gattcgtgag gctttgactc aaacccacac agctatcgct     420

gtaatcattg gcatc                                                      435

<210>  12
<211>  357
<212>  DNA
<213>  Artificial

<220>
<223>  An artificially synthesized nucleotide sequence

<400>  12
catgagatca agaaggtatt ggtgccaggc tgccacggca gcgaaccatg catcatccac      60

aggggcaagc cattccaact cgaggctgtt ttcgaggcaa accaaaacac caagaccgta     120

aagatcgaga tcaaggcaag catcgatggc ctcgaggtcg atgttcctgg catcgatcct     180

aatgcctgcc actacatgaa gtgcccactc gttaagggcc aacagtacga tatcaagtac     240

acctggaatg tgcctaagat cgccccaaag agcgagaacg ttgtggttac cgtgaaggtg     300

atgggcgatg atggcgttct cgcctgcgct atcgctaccc acgcaaagat cagggat        357

<210>  13
<211>  387
<212>  DNA
<213>  Artificial

<220>
<223>  An artificially synthesized nucleotide sequence

<400>  13
gatcaagtgg atgttaagga ttgcgccaac aatgagatca agaaggtaat ggtggatggc      60

tgccacggca gcgatccatg catcatccac aggggcaagc cattcaccct cgaggctctc     120

ttcgatgcaa accaaaacac caagaccgca aagatcgaga tcaaggcaag cctcgatggc     180

ctcgagatcg atgttcctgg catcgatacc aatgcctgcc acttcgtgaa gtgcccactc     240

gttaagggcc aacagtacga tatcaagtac acctggaatg tgcctaagat cgccccaaag     300

agcgagaacg ttgtggttac cgtgaagctc atcggcgata atggcgttct cgcctgcgct     360

atcgctaccc acggcaagat cagggat                                         387

<210>  14
<211>  129
<212>  PRT
<213>  Artificial

<220>
<223>  An artificially synthesized peptide sequence

<400>  14

Asp Gln Val Asp Val Lys Asp Ser Ala Asn Asn Glu Ile Lys Lys Val
1               5                   10                  15

Met Val Asp Gly Ser His Gly Ser Asp Pro Ser Ile Ile His Arg Gly
            20                  25                  30

Lys Pro Phe Thr Leu Glu Ala Leu Phe Asp Ala Asn Gln Asn Thr Lys
        35                  40                  45

Thr Ala Lys Ile Glu Ile Lys Ala Ser Leu Asp Gly Leu Glu Val Asp
    50                  55                  60

Val Pro Gly Ile Asp Thr Asn Ala Ser His Phe Val Lys Ser Pro Leu
65                  70                  75                  80

Val Lys Gly Gln Gln Tyr Asp Ile Lys Tyr Thr Trp Asn Val Pro Lys
                85                  90                  95

Ile Ala Pro Lys Ser Glu Asn Val Val Val Thr Val Lys Leu Ile Gly
            100                 105                 110

Asp Asn Gly Val Leu Ala Ser Ala Ile Ala Thr His Gly Lys Ile Arg
        115                 120                 125

Asp

<210>  15
<211>  129
<212>  PRT
<213>  Artificial

<220>
<223>  An artificially synthesized peptide sequence

<400>  15

Asp Gln Val Asp Val Lys Asp Ser Ala Asn Asn Glu Ile Lys Lys Val
1               5                   10                  15

Met Val Asp Gly Cys His Gly Ser Asp Pro Cys Ile Ile His Arg Gly
            20                  25                  30

Lys Pro Phe Thr Leu Glu Ala Leu Phe Asp Ala Asn Gln Asn Thr Lys
        35                  40                  45

Thr Ala Lys Ile Glu Ile Lys Ala Ser Leu Asp Gly Leu Glu Val Asp
    50                  55                  60

Val Pro Gly Ile Asp Thr Asn Ala Cys His Phe Val Lys Cys Pro Leu
65                  70                  75                  80

Val Lys Gly Gln Gln Tyr Asp Ile Lys Tyr Thr Trp Asn Val Pro Lys
                85                  90                  95

Ile Ala Pro Lys Ser Glu Asn Val Val Val Thr Val Lys Leu Ile Gly
            100                 105                 110

Asp Asn Gly Val Leu Ala Ser Ala Ile Ala Thr His Gly Lys Ile Arg
        115                 120                 125

Asp

<210>  16
<211>  129
<212>  PRT
<213>  Artificial

<220>
<223>  An artificially synthesized peptide sequence

<400>  16

Asp Gln Val Asp Val Lys Asp Cys Ala Asn Asn Glu Ile Lys Lys Val
1               5                   10                  15

Met Val Asp Gly Ser His Gly Ser Asp Pro Ser Ile Ile His Arg Gly
            20                  25                  30

Lys Pro Phe Thr Leu Glu Ala Leu Phe Asp Ala Asn Gln Asn Thr Lys
        35                  40                  45

Thr Ala Lys Ile Glu Ile Lys Ala Ser Leu Asp Gly Leu Glu Val Asp
    50                  55                  60

Val Pro Gly Ile Asp Thr Asn Ala Ser His Phe Val Lys Ser Pro Leu
65                  70                  75                  80

Val Lys Gly Gln Gln Tyr Asp Ile Lys Tyr Thr Trp Asn Val Pro Lys
                85                  90                  95

Ile Ala Pro Lys Ser Glu Asn Val Val Val Thr Val Lys Leu Ile Gly
            100                 105                 110

Asp Asn Gly Val Leu Ala Cys Ala Ile Ala Thr His Gly Lys Ile Arg
        115                 120                 125

Asp



<210>  17
<211>  24
<212>  PRT
<213>  Artificial

<220>
<223>  An artificially synthesized peptide sequence

<400>  17

Met Ala Ser Ser Val Phe Ser Arg Phe Ser Ile Tyr Phe Cys Val Leu
1               5                   10                  15

Leu Leu Cys His Gly Ser Met Ala
            20


<210>  18
<211>  24

<212>  PRT
<213>  Artificial

<220>
<223>  An artificially synthesized peptide sequence

<400>  18

Met Ala Ser Ile Asn Arg Pro Ile Val Phe Phe Thr Val Cys Leu Phe
1               5                   10                  15

Leu Leu Cys Asp Gly Ser Leu Ala
            20

<210>  19
<211>  23
<212>  PRT
<213>  Artificial

<220>
<223>  An artificially synthesized peptide sequence

<400>  19

Met Ala Ser Lys Val Val Phe Phe Ala Ala Ala Leu Met Ala Ala Met
1               5                   10                  15

Val Ala Ile Ser Gly Ala Gln
            20

<210>  20
<211>  4
<212>  PRT
<213>  Artificial

<220>
<223>  An artificially synthesized peptide sequence

<400>  20

Lys Asp Glu Leu
1

<210>  21
<211>  6
<212>  PRT
<213>  Artificial

<220>
<223>  An artificially synthesized peptide sequence

<400>  21

Ser Glu Lys Asp Glu Leu
1               5

<210>  22
<211>  4
<212>  PRT
<213>  Artificial

<220>
<223>  An artificially synthesized peptide sequence

<400>  22

His Asp Glu Leu
1

SEQUENCE LISTING

<110>  NATIONAL INSTITUTE OF AGROBIOLOGICAL SCIENCES

<120>  RICE CONTAINING MITE EPITOPE PEPTIDE

<130>  MOA-A0702P

<150> JP 2007-266864
<151> 2007-10-12

<160>  22

<170>  PatentIn version 3.4

<210>  1
<211>  669
<212>  DNA
<213>  Dermatophagoides pteronyssinus

<400>  1
actaacgcct gcagtatcaa tggaaatgct ccagctgaaa tcgatttgcg acaaatgcga      60

actgtcactc ccattcgtat gcaaggaggc tgtggttcat gttgggcttt ctctggtgtt     120

gccgcaactg aatcagctta tttggcttac cgtaatcaat cattggatct tgctgaacaa     180

gaattagtcg attgtgcttc ccaacacggt tgtcatggtg ataccattcc acgtggtatt     240

gaatacatcc aacataatgg tgtcgtccaa gaaagctact atcgatacgt tgcacgagaa     300

caatcatgcc gacgaccaaa tgcacaacgt ttcggtatct caaactattg ccaaatttac     360

ccaccaaatg caaacaaaat tcgtgaagct ttggctcaaa cccacagcgc tattgccgtc     420

attattggca tcaaagattt agacgcattc cgtcattatg atggccgaac aatcattcaa     480

cgcgataatg gttaccaacc aaactatcac gctgtcaaca ttgttggcta cagtaacgca     540

caaggtgtcg attattggat cgtacgaaac agttgggata ccaattgggg tgataatggt     600

tacggttatt ttgctgccaa catcgatttg atgatgattg aagaatatcc atatgttgtc     660

attctctaa                                                             669


<210>  2
<211>  222
<212>  PRT
<213>  Dermatophagoides pteronyssinus

<400>  2

Thr Asn Ala Cys Ser Ile Asn Gly Asn Ala Pro Ala Glu Ile Asp Leu
1               5                   10                  15


Arg Gln Met Arg Thr Val Thr Pro Ile Arg Met Gln Gly Gly Cys Gly
            20                  25                  30

Ser Cys Trp Ala Phe Ser Gly Val Ala Ala Thr Glu Ser Ala Tyr Leu
        35                  40                  45

Ala Tyr Arg Asn Gln Ser Leu Asp Leu Ala Glu Gln Glu Leu Val Asp
    50                  55                  60

Cys Ala Ser Gln His Gly Cys His Gly Asp Thr Ile Pro Arg Gly Ile
65                  70                  75                  80

Glu Tyr Ile Gln His Asn Gly Val Val Gln Glu Ser Tyr Tyr Arg Tyr
                85                  90                  95

Val Ala Arg Glu Gln Ser Cys Arg Arg Pro Asn Ala Gln Arg Phe Gly
            100                 105                 110

Ile Ser Asn Tyr Cys Gln Ile Tyr Pro Pro Asn Ala Asn Lys Ile Arg
        115                 120                 125

Glu Ala Leu Ala Gln Thr His Ser Ala Ile Ala Val Ile Ile Gly Ile
    130                 135                 140

Lys Asp Leu Asp Ala Phe Arg His Tyr Asp Gly Arg Thr Ile Ile Gln
145                 150                 155                 160

Arg Asp Asn Gly Tyr Gln Pro Asn Tyr His Ala Val Asn Ile Val Gly
                165                 170                 175

Tyr Ser Asn Ala Gln Gly Val Asp Tyr Trp Ile Val Arg Asn Ser Trp
            180                 185                 190

Asp Thr Asn Trp Gly Asp Asn Gly Tyr Gly Tyr Phe Ala Ala Asn Ile
        195                 200                 205

Asp Leu Met Met Ile Glu Glu Tyr Pro Tyr Val Val Ile Leu
    210                 215                 220

<210>  3
<211>  390
<212>  DNA
<213>  Dermatophagoides pteronyssinus

<400>  3
gatcaagtcg atgtcaaaga ttgtgccaat catgaaatca aaaaagtttt ggtaccagga      60

tgccatggtt cagaaccatg tatcattcat cgtggtaaac cattccaatt ggaagccgtt     120

ttcgaagcca accaaaacac aaaaacggct aaaattgaaa tcaaagcctc aatcgatggt     180

ttagaagttg atgttcccgg tatcgatcca aatgcatgcc attacatgaa atgcccattg     240

gttaaaggac aacaatatga tattaaatat acatggaatg ttccgaaaat tgcaccaaaa    300

tctgaaaatg ttgtcgtcac tgttaaagtt atgggtgatg atggtgtttt ggcctgtgct    360

attgctactc atgctaaaat ccgcgattaa                                      390

<210>  4
<211>  129
<212>  PRT
<213>  Dermatophagoides pteronyssinus

<400>  4

Asp Gln Val Asp Val Lys Asp Cys Ala Asn His Glu Ile Lys Lys Val
1               5                   10                  15

Leu Val Pro Gly Cys His Gly Ser Glu Pro Cys Ile Ile His Arg Gly
            20                  25                  30

Lys Pro Phe Gln Leu Glu Ala Val Phe Glu Ala Asn Gln Asn Thr Lys
        35                  40                  45

Thr Ala Lys Ile Glu Ile Lys Ala Ser Ile Asp Gly Leu Glu Val Asp
    50                  55                  60

Val Pro Gly Ile Asp Pro Asn Ala Cys His Tyr Met Lys Cys Pro Leu
65                  70                  75                  80

Val Lys Gly Gln Gln Tyr Asp Ile Lys Tyr Thr Trp Asn Val Pro Lys
                85                  90                  95

Ile Ala Pro Lys Ser Glu Asn Val Val Val Thr Val Lys Val Met Gly
            100                 105                 110

Asp Asp Gly Val Leu Ala Cys Ala Ile Ala Thr His Ala Lys Ile Arg
        115                 120                 125

Asp

<210>  5
<211>  672
<212>  DNA
<213>  Dermatophagoides farinae

<400>  5
acaagcgctt gccgtatcaa ttcggttaac gttccatcgg aattggattt acgatcactg     60

cgaactgtca ctccaatccg tatgcaagga ggctgtggtt catgttgggc tttctctggt    120

gtcgccgcaa ctgaatcagc ttatttggcc taccgtaaca cgtctttgga tctttctgaa    180

caggaactcg tcgattgcgc atctcaacac ggatgtcacg gcgatacaat accaagaggc    240

atcgaataca tccaacaaaa tggtgtcgtt gaagaaagaa gctatccata cgttgcacga    300

gaacaacaat gccgacgacc aaattcgcaa cattacggta tctcaaacta ctgccaaatt    360

tatccaccag atgtgaaaca aatccgtgaa gctttgactc aaacacacac agctattgcc    420

gtcattattg gcattaaaga tttgagagct tttcaacatt atgatggacg aacaatcatt    480

caacatgaca atggttatca accaaactat catgccgtca acattgtcgg ttacggaagt    540

acacaaggcg tcgattattg gatcgtacga aacagttggg atactacctg gggtgatagc    600

ggatacggat atttccaagc cggaaacaac ctcatgatga tcgaacaata tccatatgtt    660

gtaatcatgt ga                                                        672

<210>  6
<211>  223
<212>  PRT
<213>  Dermatophagoides farinae

<400>  6

<210>  7
<211>  390
<212>  DNA
<213>  Dermatophagoides farinae

<400>  7
gatcaagtcg atgttaaaga ttgtgccaac aatgaaatca aaaaagtaat ggtcgatggt      60

tgccatggtt ctgatccatg catcatccat cgtggtaaac cattcacttt ggaagcctta     120

ttcgatgcca accaaaacac taaaaccgct aaaattgaaa tcaaagccag cctcgatggt     180

cttgaaattg atgttcccgg tatcgatacc aatgcttgcc attttatgaa atgtccattg     240

gttaaaggtc aacaatatga tgccaaatat acatggaatg tgccgaaaat tgcaccaaaa     300

tctgaaaacg ttgtcgttac agtcaaactt gttggtgata atggtgtttt ggcttgcgct     360

attgctaccc atggtaaaat ccgtgattaa                                      390

<210>  8
<211>  129
<212>  PRT
<213>  Dermatophagoides farinae

<400>  8

Asp Gln Val Asp Val Lys Asp Cys Ala Asn Asn Glu Ile Lys Lys Val
1               5                   10                  15

Met Val Asp Gly Cys His Gly Ser Asp Pro Cys Ile Ile His Arg Gly
            20                  25                  30

Lys Pro Phe Thr Leu Glu Ala Leu Phe Asp Ala Asn Gln Asn Ser Lys
        35                  40                  45

Thr Ala Lys Ile Glu Ile Lys Ala Ser Leu Asp Gly Leu Glu Ile Asp
    50                  55                  60

Val Pro Gly Ile Asp Thr Asn Ala Cys His Phe Val Lys Cys Pro Leu
65                  70                  75                  80

Val Lys Gly Gln Gln Tyr Asp Ile Lys Tyr Thr Trp Asn Val Pro Lys
                85                  90                  95

Ile Ala Pro Lys Ser Glu Asn Val Val Val Thr Val Lys Leu Ile Gly
            100                 105                 110

Asp Asn Gly Val Leu Ala Cys Ala Ile Ala Thr His Gly Lys Ile Arg
        115                 120                 125

Asp

<210>  9
<211>  329
<212>  DNA
<213>  Artificial

<220>
<223>  An artificially synthesized nucleotide sequence

<400>  9
ccatgggatc agcttatttg gcttaccgta atcaatcatt ggatcttgct gagcaggaat      60

tggtagattg tgcttcccaa cacggatgtc atggtgatac cattccacgt ggtattgaat     120

acatccaaca taatggagtt gtacaggaga gctactatcg ctacgttgca cgtgaacaat     180

catgccgccg tccaaatgca caacgtttcg gaatctcaaa ctattgccag atttacccac     240

caaatgtaaa caagattcgt gaggctttgg cacaaaccca cagcgctatt gcagttatta     300

ttggcatcaa ggacgagttg taagagctc                                       329

<210>  10
<211>  106
<212>  PRT
<213>  Artificial

<220>
<223>  An artificially synthesized peptide sequence

<400>  10

Met Gly Ser Ala Tyr Leu Ala Tyr Arg Asn Gln Ser Leu Asp Leu Ala
1               5                   10                  15

Glu Gln Glu Leu Val Asp Cys Ala Ser Gln His Gly Cys His Gly Asp
            20                  25                  30

Thr Ile Pro Arg Gly Ile Glu Tyr Ile Gln His Asn Gly Val Val Gln
        35                  40                  45

Glu Ser Tyr Tyr Arg Tyr Val Ala Arg Glu Gln Ser Cys Arg Arg Pro
    50                  55                  60

Asn Ala Gln Arg Phe Gly Ile Ser Asn Tyr Cys Gln Ile Tyr Pro Pro
65                  70                  75                  80

Asn Val Asn Lys Ile Arg Glu Ala Leu Ala Gln Thr His Ser Ala Ile
                85                  90                  95

Ala Val Ile Ile Gly Ile Lys Asp Glu Leu
            100                 105

<210>  11
<211>  435
<212>  DNA
<213>  Artificial

<220>
<223>  An artificially synthesized nucleotide sequence

<400>  11
actagtgcat gcaggatcaa cagcgttaat gtaccatctg agcttgatct cagaagtttg      60

aggacagtta ctccaatccg tatgcaggga ggctgcggct ctgcttgggc attcagcggc     120

gtagcagcta ccgaatcagc ttatttggct taccgtaata cttcattgga tctttctgag     180

caggaattgg tagattgtgc ttcccaacac ggatgtcatg gtgataccat tccacgtggt     240

attgaataca tccaacaaaa tggagttgta gaggagagaa gctatcccta cgttgcacgt     300

gaacaacaat gccgccgtcc aaattcacaa cattacggaa tctcaaacta ttgccagatt     360

tacccaccag atgtaaagca gattcgtgag gctttgactc aaacccacac agctatcgct     420

gtaatcattg gcatc                                                      435

<210>  12
<211>  357
<212>  DNA
<213>  Artificial

<220>
<223>  An artificially synthesized nucleotide sequence

<400>  12
catgagatca agaaggtatt ggtgccaggc tgccacggca gcgaaccatg catcatccac      60

aggggcaagc cattccaact cgaggctgtt ttcgaggcaa accaaaacac caagaccgta 120

aagatcgaga tcaaggcaag catcgatggc ctcgaggtcg atgttcctgg catcgatcct 180

aatgcctgcc actacatgaa gtgcccactc gttaagggcc aacagtacga tatcaagtac 240

acctggaatg tgcctaagat cgccccaaag agcgagaacg ttgtggttac cgtgaaggtg 300

atgggcgatg atggcgttct cgcctgcgct atcgctaccc acgcaaagat cagggat 357

<210> 13
<211> 387
<212> DNA
<213> Artificial

<220>
<223> An artificially synthesized nucleotide sequence

<400> 13
gatcaagtgg atgttaagga ttgcgccaac aatgagatca agaaggtaat ggtggatggc 60

tgccacggca gcgatccatg catcatccac aggggcaagc cattcaccct cgaggctctc 120

ttcgatgcaa accaaaacac caagaccgca aagatcgaga tcaaggcaag cctcgatggc 180

ctcgagatcg atgttcctgg catcgatacc aatgcctgcc acttcgtgaa gtgcccactc 240

gttaagggcc aacagtacga tatcaagtac acctggaatg tgcctaagat cgccccaaag 300

agcgagaacg ttgtggttac cgtgaagctc atcggcgata atggcgttct cgcctgcgct 360

atcgctaccc acggcaagat cagggat 387

<210> 14
<211> 129
<212> PRT
<213> Artificial

<220>
<223> An artificially synthesized peptide sequence

<400> 14

Asp Gln Val Asp Val Lys Asp Ser Ala Asn Asn Glu Ile Lys Lys Val
1               5                   10                  15

Met Val Asp Gly Ser His Gly Ser Asp Pro Ser Ile Ile His Arg Gly
            20                  25                  30

Lys Pro Phe Thr Leu Glu Ala Leu Phe Asp Ala Asn Gln Asn Thr Lys
        35                  40                  45

Thr Ala Lys Ile Glu Ile Lys Ala Ser Leu Asp Gly Leu Glu Val Asp
    50                  55                  60

Val Pro Gly Ile Asp Thr Asn Ala Ser His Phe Val Lys Ser Pro Leu
65                  70                  75                  80

Val Lys Gly Gln Gln Tyr Asp Ile Lys Tyr Thr Trp Asn Val Pro Lys
                85                  90                  95

Ile Ala Pro Lys Ser Glu Asn Val Val Val Thr Val Lys Leu Ile Gly
            100                 105                 110

Asp Asn Gly Val Leu Ala Ser Ala Ile Ala Thr His Gly Lys Ile Arg
        115                 120                 125

Asp

<210>  15
<211>  129
<212>  PRT
<213>  Artificial

<220>
<223>  An artificially synthesized peptide sequence

<400>  15

Asp Gln Val Asp Val Lys Asp Ser Ala Asn Asn Glu Ile Lys Lys Val
1               5                   10                  15

Met Val Asp Gly Cys His Gly Ser Asp Pro Cys Ile Ile His Arg Gly
            20                  25                  30

Lys Pro Phe Thr Leu Glu Ala Leu Phe Asp Ala Asn Gln Asn Thr Lys
        35                  40                  45

Thr Ala Lys Ile Glu Ile Lys Ala Ser Leu Asp Gly Leu Glu Val Asp
    50                  55                  60

Val Pro Gly Ile Asp Thr Asn Ala Cys His Phe Val Lys Cys Pro Leu
65                  70                  75                  80

Val Lys Gly Gln Gln Tyr Asp Ile Lys Tyr Thr Trp Asn Val Pro Lys
                85                  90                  95

Ile Ala Pro Lys Ser Glu Asn Val Val Val Thr Val Lys Leu Ile Gly
            100                 105                 110

Asp Asn Gly Val Leu Ala Ser Ala Ile Ala Thr His Gly Lys Ile Arg
        115                 120                 125

Asp

<210>  16
<211>  129
<212>  PRT
<213>  Artificial

<220>
<223>  An artificially synthesized peptide sequence

<400>  16

Asp Gln Val Asp Val Lys Asp Cys Ala Asn Asn Glu Ile Lys Lys Val
1               5                   10                  15

Met Val Asp Gly Ser His Gly Ser Asp Pro Ser Ile Ile His Arg Gly
            20                  25                  30

Lys Pro Phe Thr Leu Glu Ala Leu Phe Asp Ala Asn Gln Asn Thr Lys
        35                  40                  45

Thr Ala Lys Ile Glu Ile Lys Ala Ser Leu Asp Gly Leu Glu Val Asp
    50                  55                  60

Val Pro Gly Ile Asp Thr Asn Ala Ser His Phe Val Lys Ser Pro Leu
65                  70                  75                  80

Val Lys Gly Gln Gln Tyr Asp Ile Lys Tyr Thr Trp Asn Val Pro Lys
                85                  90                  95

Ile Ala Pro Lys Ser Glu Asn Val Val Val Thr Val Lys Leu Ile Gly
            100                 105                 110

Asp Asn Gly Val Leu Ala Cys Ala Ile Ala Thr His Gly Lys Ile Arg
        115                 120                 125

Asp

<210>  17
<211>  24
<212>  PRT
<213>  Artificial

<220>
<223>  An artificially synthesized peptide sequence

<400>  17

Met Ala Ser Ser Val Phe Ser Arg Phe Ser Ile Tyr Phe Cys Val Leu
1               5                   10                  15

Leu Leu Cys His Gly Ser Met Ala
            20

<210>  18
<211>  24
<212>  PRT
<213>  Artificial

<220>
<223>  An artificially synthesized peptide sequence

<400>  18

Met Ala Ser Ile Asn Arg Pro Ile Val Phe Phe Thr Val Cys Leu Phe
1               5                   10                  15

Leu Leu Cys Asp Gly Ser Leu Ala
            20

<210>  19
<211>  23
<212>  PRT
<213>  Artificial

<220>
<223>  An artificially synthesized peptide sequence

<400>  19

Met Ala Ser Lys Val Val Phe Phe Ala Ala Ala Leu Met Ala Ala Met
1               5                   10                  15

Val Ala Ile Ser Gly Ala Gln
            20

<210>  20
<211>  4
<212>  PRT
<213>  Artificial

<220>
<223>  An artificially synthesized peptide sequence

<400>  20

Lys Asp Glu Leu
1

<210>  21
<211>  6

<212>  PRT
<213>  Artificial

<220>
<223>  An artificially synthesized peptide sequence

<400>  21

Ser Glu Lys Asp Glu Leu
1               5


<210>  22
<211>  4
<212>  PRT
<213>  Artificial

<220>
<223>  An artificially synthesized peptide sequence

<400>  22

His Asp Glu Leu
1